(19) **Europäisches Patentamt**
**European Patent Office**
**Office européen des brevets**

(11) **EP 2 210 108 B1**

(12) **EUROPEAN PATENT SPECIFICATION**

(45) Date of publication and mention
of the grant of the patent:
**07.12.2011 Bulletin 2011/49**

(51) Int Cl.:
**G01N 33/68** (2006.01)

(21) Application number: **08837800.5**

(22) Date of filing: **10.10.2008**

(86) International application number:
**PCT/US2008/079553**

(87) International publication number:
**WO 2009/049189 (16.04.2009 Gazette 2009/16)**

(54) **METHODS FOR DETECTING MAJOR ADVERSE CARDIOVASCULAR AND CEREBROVASCULAR EVENTS**

VERFAHREN ZUM NACHWEIS GRÖSSERER UNGÜNSTIGER KARDIOVASKULÄRER UND ZEREBROVASKULÄRER EREIGNISSE

PROCÉDÉS POUR DÉTECTER DES ÉVÉNEMENTS CARDIOVASCULAIRES ET CÉRÉBROVASCULAIRES PRÉJUDICIABLES MAJEURS

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **10.10.2007 US 998563 P**
**11.10.2007 US 998756 P**

(43) Date of publication of application:
**28.07.2010 Bulletin 2010/30**

(73) Proprietor: **BG Medicine, Inc.**
**Waltham, MA 02451 (US)**

(72) Inventors:
• **MUNTENDAM, Pieter**
**Boxford, MA 01921 (US)**
• **BALASUBRAMANIAN, Rajalakshmi**
**Wayland, MA 01778 (US)**
• **MYERS, Rene**
**Framingham, MA 01701 (US)**

(74) Representative: **Chapman, Paul William et al**
**Kilburn & Strode LLP**
**20 Red Lion Street**
**London**
**WC1R 4PJ (GB)**

(56) References cited:
**WO-A-2007/124439      US-A1- 2007 003 981**

• **NUNEZ JULIO ET AL: "Carbohydrate antigen 125: an emerging prognostic risk factor in acute heart failure?" HEART (LONDON), vol. 93, no. 6, June 2007 (2007-06), pages 716-721, XP008101461 ISSN: 1355-6037**
• **JOVIN T G ET AL: "High titers of CA-125 may be associated with recurrent ischemic strokes in patients with cancer" NEUROLOGY, vol. 64, no. 11, June 2005 (2005-06), pages 1944-1945, XP002513202 ISSN: 0028-3878**
• **VASAN RAMACHANDRAN S: "Biomarkers of cardiovascular disease - Molecular basis and practical considerations" CIRCULATION, AMERICAN HEART ASSOCIATION, DALLAS, TX, vol. 113, no. 19, 1 May 2006 (2006-05-01), pages 2335-2362, XP002464236 ISSN: 0009-7322 cited in the application**
• **MITCHELL A M ET AL: "Multimarker Panel to Rule Out Acute Coronary Syndromes in Low-risk Patients" ACADEMIC EMERGENCY MEDICINE 200607 US, vol. 13, no. 7, July 2006 (2006-07), pages 803-806, XP002513203 ISSN: 1069-6563**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present teachings relate to methods for predicting if a human will suffer a major adverse cardiovascular or cerebrovascular event, or MACCE. More specifically, the present teachings relate to methods for screening an individual for being at risk of having or developing a major adverse cardiovascular or cerebrovascular event by using one or more analytes.

BACKGROUND

Heart attack is the single leading cause of death (see www.americanheart.org). One of every 5 deaths in the United States results from a heart attack. In 2004, there were 452,327 deaths in the United States due to heart attack resulting from approximately 1,200,000 new and recurrent cardiovascular attacks.

Stroke is the third leading cause of death in the United States (see www.americanheart.org). Stroke killed 150,147 people in 2004 resulting from approximately 700,000 new and recurrent cerebrovascular events. Stroke is a leading cause of serious, long-term disability in the United States. About 5,700,000 stroke survivors are alive today in the United States. 2,400,000 are males and 3,300,000 are females.

Both heart attack and certain types of stroke can result from the rupture of vulnerable atherosclerotic plaque (Naghavi, et al., Circulation 108: 1664-72 & 108:1772-8, 2003). At present, the risk of having a heart attack or stroke is assessed in the general population by considering certain clinical and biochemical risk factors (Wilson et al., Circulation 97:1837-47, 1998; ATP III, JAMA 285:2486-97, 2001), but these characteristics do not fully explain cardiovascular risk (Khot, et al., JAMA 290:898-904, 2003; Greenland, et al., JAMA 290:891-7, 2003).

If the ability to predict the future occurrence of heart attack or stroke could be improved, individuals with such risk could be targeted for preventative measures and the overall incidence of these leading causes of death could be reduced.

Measurement of multiple proteins and metabolites in the blood of an individual offers the prospect of a "window" into that individual's biochemical status and might provide a better indication of the status of his or her cardiovascular system and the likelihood of that subject experiencing a future heart attack or stroke (Vasan, Circulation 113:2335-62, 2006). With this rationale, we conducted a study with the objective of discovering a molecular biomarker profile (e.g., a set of proteins, a set of metabolites, a set of proteins and metabolites, or a set of other analytes which can include proteins and/or metabolites), in blood or blood plasma and associated algorithm that predicts a near-term major adverse cardiovascular or cerebrovascular event (MACCE).

US 2007/003981 discloses panels of biomarkers for identifying ACS (acute coronary syndrome) in at risk patients. These panels may include, *infer alia,* glutathione S-transferase, tissue factor and alpha-fetoprotein and some of these panels may include five or more biomarkers.

Thus, there remains a need for diagnostic methods for predicting major acute cardiac events. In particular, reliable and cost-effective methods and compositions are needed to allow for diagnosis and/or prediction of major adverse cardiovascular and cerebrovascular events.

SUMMARY OF THE INVENTION

The present invention provides a method of assessing the probability of a major adverse cardiovascular or cerebrovascular event in a human, the method comprising:

measuring a concentration, in a blood-based sample of a human, of a set of analytes comprising alpha-fetoprotein, cancer antigen 125, glutathione S-transferase, and tissue factor;
determining an index for the set of analytes; and
identifying the human as having an increased likelihood of a major adverse cardiovascular or cerebrovascular event if the index is greater than zero, or a decreased likelihood of a major adverse cardiovascular or cerebrovascular event if the index is less than or equal to zero,
wherein determining an index for the set of analytes comprises:

standardizing the measured concentration of each analyte to obtain a standardized concentration;
multiplying the standardized concentration of each analyte by an analyte constant to obtain an analyte value; and
summing the analyte value of each analyte to obtain the index.

According to various embodiments, the set of analytes also can include CD40, fibrinogen, IL-3, IL-8, SGOT, and von Willebrand factor.

According to various embodiments, standardizing the measured concentration can include subtracting from the measured concentration a population average value to obtain a result and then dividing the result by a standard deviation of the population average value.

According to various embodiments, the blood-based sample can be serum or plasma.

In further embodiments the method also can include transmitting, displaying, storing or outputting the index to a user interface device, a computer readable storage medium, or a local or remote computer system.

BRIEF DESCRIPTION OF THE DRAWINGS

The foregoing and other objects, features and advantages of the present teachings described above will be more fully understood from the following description of various illustrative embodiments, when read together with the accompanying drawings. In the drawings, like reference characters generally refer to the same parts throughout the different views. It should be understood that the drawings described below are for illustration purposes only. The drawings are not intended to limit the scope of the present teachings in any way.

Figure 1 is a Receiver Operating Characteristic (ROC) curve depicting the prediction for the occurrence of a MACCE within two years using the levels of the top 20 analytes from Table 5 in a plasma sample according to an exemplary embodiment of the present teachings.

Figure 2 is a Receiver Operating Characteristic (ROC) curve depicting the prediction for the occurrence of a MACCE within two years using the levels of the top 10 protein analytes from Table 6 in a plasma sample according to an exemplary embodiment of the present teachings.

DETAILED DESCRIPTION

Analytes have been identified that are predictive of major adverse cardiovascular or cerebrovascular events. When one or more of these analytes are present in a body fluid sample from an individual in amounts different than those in a standard, they can be indicative that the individual is at risk of having or developing a major adverse cardiovascular or cerebrovascular event.

Seven hundred twenty-three (723) analytes were identified. Certain methods according to the present teachings utilize two or more of these analytes to predict a major adverse cardiovascular or cerebrovascular event. Specifically, in these methods, a sample from a subject can be screened to determine whether the sample contains levels of two or more of each of these seven hundred twenty-three (723) analytes that are different from a standard sample. If the sample contains an amount of each of two or more of these analytes that is different from the amount of these analytes in a standard, the screen is considered a "positive screen" (i.e., the individual is at risk of suffering a major adverse cardiovascular or cerebrovascular event). Greater sensitivity and specificity in classifying major adverse cardiovascular or cerebrovascular event samples can be obtained, typically, by using a greater number of the analytes. The samples potentially containing these analytes can be drawn from multiple biological samples (e.g., body fluids, tissue, cells) obtained from multiple sources (e.g., whole blood, blood plasma, blood serum, urine, cerebrospinal fluid, epithelial cells, and endothelial cells). It should be understood that all possible combinations of the seven hundred twenty-three (723) analytes disclosed herein (and not just the fifty (50) analytes identified in Table 5) can be used in methods according to the present teachings.

Using the methodology described more fully in Example 1, the seven hundred twenty-three (723) analytes were identified. Briefly, through a specific analytical classification protocol, a set of seven hundred twenty three (723) analytes were analyzed and spectral peaks were obtained. Appendices 1 through 4 provides specific molecules comprising the seven hundred twenty-three (723) analyzed analytes. These peaks characterize specific molecules. Utilizing these peaks (which include all 723 analytes), fifty peaks were identified (as well as other preferred peaks as described below). Insofar as peaks characterize molecules, identifying fifty peaks from among a much larger number of peaks means that the analytes are chosen from a group of molecules including more than the fifty molecules corresponding to the fifty analytes. The analytes (i.e., the molecules) may be any type of molecule. The analytes (molecules) include, but are not limited to, proteins, peptides, amino acids, lipids, steroids, nucleic acids, metabolites and elements. Table 5 provides specific molecules comprising the fifty peaks (i.e., identifying the chosen analytes). The second column of Table 5 rank-orders the peaks by weight. Accordingly, the highest weight peak is ranked number 1 and the lowest weight peak is ranked number 50.

Now that the analytes (i.e., any of the seven hundred twenty-three identified analytes or any of the preferred fifty analytes) are known, they can be used to screen an individual to determine whether the amount or absolute concentration of each of two or more of these analytes in a sample from the individual is different from the amount of each of the two or more analytes from a standard, to determine the individual's relative concentration of each of the two or more analytes in the sample compared to a standard, and classifying the individual, to a certain specificity and sensitivity, as having or being at risk of developing a major adverse cardiovascular or cerebrovascular event. Of course the measured amount or concentration of an analyte can be standardized prior to the comparison. Based on the number of analytes examined, the desired sensitivity and specificity of the assay can be chosen. The standard can be an actual sample or previously-generated empirical data. The standard can be obtained from a known normal person. The known normal person can be a healthy person and can have a predetermined dietary intake for a predetermined time before sampling. Moreover, the sample can be obtained from a known normal person of the same sex as the subject. Alternatively, the analytes could be compared to those of a known major adverse cardiovascular or cerebrovascular event subject, in which case the similarity between the two samples, or the relative concentration of the analyte compared to a standard, would be examined. Various techniques and/or kits can be used by a medical professional for screening subject samples in order to determine the level and/or amount of a particular analyte in a subject sample. Examples of such assays are described below and include, but are not limited to, an immunoassay, mass spectroscopy, chromatography, a chemical analysis, a colorimetric assay, a spectrophotometric analysis, an electrochemical analysis, and nuclear magnetic resonance. Additionally, such assays can be performed on any biological sample including whole blood, blood plasma, blood serum, cerebrospinal fluid, saliva, urine, seminal fluid, breast nipple aspirate, pancreatic fluid, and combinations thereof. These assays are chosen based on which are best suited to detect a particular analyte as well as which are best suited for use with a particular biological sample. Accordingly, multiple assays can be used to detect the desired analytes, and samples can be analyzed from one or more sources.

An analyte can be detected and/or quantified by using one or more separation methods. For example, suitable separation methods may include a mass spectrometry method, such as electrospray ionization mass spectrometry (ESI-MS), ESI-MS/MS, ESI-MS/$(MS)^n$ (n is an integer greater than zero), matrix-assisted laser desorption ionization time-of-flight mass spectrometry (MALDI-TOF-MS), surface-enhanced laser desorption/ionization time-of-flight mass spectrometry (SELDI-TOF-MS), desorption/ionization on silicon (DIOS), secondary ion mass spectrometry (SIMS), quadrupole time-of-flight (Q-TOF), atmospheric pressure chemical ionization mass spectrometry (APCI-MS), APCI-MS/MS, APCI-$(MS)^n$, atmospheric pressure photoionization mass spectrometry (APPI-MS), APPI-MS/MS, and APPI-$(MS)^n$. Other mass spectrometry methods may include, inter alia, quadrupole, fourier transform mass spectrometry (FTMS) and ion trap. Spectrometric techniques that can also be used include resonance spectroscopy and optical spectroscopy.

Other suitable separation methods include chemical extraction partitioning, column chromatography, ion exchange chromatography, hydrophobic (reverse phase) liquid chromatography, isoelectric focusing, one-dimensional polyacrylamide gel electrophoresis (PAGE), two-dimensional polyacrylamide gel electrophoresis (2D-PAGE), or other chromatographic techniques, such as thin-layer, gas or liquid chromatography, or any combination thereof. In one embodiment, the biological sample to be assayed may be fractionated prior to application of the separation method.

Tandem linking of chromatography (for example liquid chromatography ("LC")) and mass spectrometry ("MS") can be useful for detecting and quantifying one or more of the analytes. LC can be used to separate the molecules, which may include an analyte, in a sample from an individual. A small amount of the sample, dissolved in a solvent, can be injected into the injection port of the LC device, which can be kept at a high temperature. The LC column of the device contains a solid substrate that can be either polar or non-polar. Because of differing polarities of the molecules in the sample, the molecules will have differing affinities for the solid substrate in the column and will elute at different times. The stronger the affinity of the molecule to the substrate, the longer the retention time of the molecule in the column. As the molecules exit the column, they enter the mass spectrometer. The mass spectrometer ionizes the molecules. In the tandem mass spectrometry mode, if the system can be standardized properly, each compound sent into a mass spectrometer fragments into ions of various masses and abundances forming a signature pattern unique to that substance. By comparing the tandem mass spectrograph of each peak to a computerized database, the computer is usually able to identify the molecules with a high degree of certainty. Alternately, or additionally, this comparison may be carried out by human inspection. Once an identity is established, the computer integrates the area under each peak and thereby determines the relative quantity of each molecule in the mixture. To the extent any of the molecules are identified as an analyte, the amount of the analyte can be compared with the amount of the analyte from a standard to determine if there is a difference.

Analytes also can be detected and/or quantified by methods that do not require physical separation of the analytes themselves. For example, nuclear magnetic resonance (NMR) spectroscopy can be used to resolve a profile of an analyte from a complex mixture of molecules. An analogous use of NMR to classify tumors is disclosed in Hagberg, NMR Biomed. 11: 148-56 (1998), for example. Additional procedures include nucleic acid amplification technologies, which can be used to determine an analyte profile without physical separation of individual molecules. (See Stordeur et al., J. Immunol. Methods 259: 55-64 (2002) and Tan et al., Proc. Nat'1 Acad. Sci. USA 99: 11387-11392 (2002), for example.)

An analyte in a sample also can be detected and/or quantified, for example, by combining the analyte with a binding moiety capable of specifically binding the analyte. The binding moiety can include, for example, a member of a ligand-receptor pair, i.e., a pair of molecules capable of having a specific binding interaction. The binding moiety can also include, for example, a member of a specific binding pair, such as antibody-antigen, enzyme-substrate, nucleic acid-nucleic acid, protein-nucleic acid, protein-protein, or other specific binding pairs known in the art. Binding proteins may be designed which have enhanced affinity for a target. Optionally, the binding moiety may be linked with a detectable label, such as an enzymatic, fluorescent, radioactive, phosphorescent or colored particle label. The labeled complex may be detected, e.g., visually or with the aid of a spectrophotometer or other detector, and/or may be quantified.

An analyte also may be detected and/or quantified using gel electrophoresis techniques available in the art. In two-dimensional gel electrophoresis, molecules are separated first in a pH gradient gel according to their isoelectric point. The resulting gel then can be placed on a second polyacrylamide gel, and the molecules separated according to molecular weight (See, for example, O'Farrell J. Biol. Chem. 250: 4007-4021 (1975)). An analyte for major adverse cardiovascular or cerebrovascular event may be detected by first isolating molecules from a sample obtained from an individual suspected of being at risk for a major adverse cardiovascular or cerebrovascular event and then separating the molecules by two-dimensional gel electrophoresis to produce a characteristic two-dimensional gel electrophoresis pattern. The pattern may then be compared with a standard gel pattern produced by separating, under the same or similar conditions, molecules isolated from the standard (e.g., healthy or major acute cardiac event subjects). The standard gel pattern may be stored in, and retrieved from, an electronic database of electrophoresis patterns. Thus, it can be determined if the amount of the marker in the subject is different from the amount in the standard. The presence of a plurality, e.g., two to fifty, major adverse cardiovascular or cerebrovascular event analytes on the two-dimensional gel in an amount different than a known normal standard indicates a positive screen for a major adverse cardiovascular or cerebrovascular event in the individual. The assay thus permits the prediction and treatment of major adverse cardiovascular or cerebrovascular events.

An analyte also may be detected and/or quantified using any of a wide range of immunoassay techniques available in the art. For example, sandwich immunoassay format may be used to detect and/or quantify an analyte in a sample from a subject. Alternatively, conventional immuno-histochemical procedures may be used for detecting and/or quantifying the presence of an analyte in a sample using one or more labeled binding proteins.

In a sandwich immunoassay, two antibodies capable of binding an analytes generally are used, e.g., one immobilized onto a solid support, and one free in solution and labeled with a detectable chemical compound. Examples of chemical labels that may be used for the second antibody include radioisotopes, fluorescent compounds, and enzymes or other molecules that generate colored or electrochemically active products when exposed to a reactant or enzyme substrate. When a sample containing the analyte is placed in this system, the analyte binds to both the immobilized antibody and the labeled antibody, to form a "sandwich" immune complex on the support's surface. The complexed analyte is detected by washing away non-bound sample components and excess labeled antibody, and measuring the amount of labeled antibody complexed to the analyte on the support's s surface. Alternatively, the antibody free in solution, which can be labeled with a chemical moiety, for example, a hapten, may be detected by a third antibody labeled with a detectable moiety which binds the free antibody or, for example, the hapten coupled thereto.

Both the sandwich immunoassay and tissue immunohistochemical procedures are highly specific and very sensitive, provided that labels with good limits of detection are used. A detailed review of immunological assay design, theory and protocols can be found in numerous texts in the art, including Butt, W.R., Practical Immunology, ed. Marcel Dekker, New York (1984) and Harlow et al. Antibodies, A Laboratory Approach, ed. Cold Spring Harbor Laboratory (1988).

In general, immunoassay design considerations include preparation of antibodies (e.g., monoclonal or polyclonal antibodies) having sufficiently high binding specificity for the target to form a complex that can be distinguished reliably from products of nonspecific interactions. As used herein, the term "antibody" is understood to mean binding proteins, for example, antibodies or other proteins comprising an immunoglobulin variable region-like binding domain, having the appropriate binding affinities and specificities for the target. The higher the antibody binding specificity, the lower the target concentration that can be detected. As used herein, the terms "specific binding" or "binding specifically" are understood to mean that the binding moiety, for example, a binding protein, has a binding affinity for the target of greater than about $10^5$ M$^{-1}$, more preferably greater than about $10^7$ M$^{-1}$.

0] Antibodies to an isolated target analyte which are useful in assays for predicting a major adverse cardiovascular or cerebrovascular event in an individual may be generated using standard immunological procedures well known and described in the art. See, for example Practical Immunology, supra. Briefly, an isolated analyte can be used to raise antibodies in a xenogeneic host, such as a mouse, goat or other suitable mammal. The analyte can be combined with a suitable adjuvant capable of enhancing antibody production in the host, and can be injected into the host, for example, by intraperitoneal administration. Any adjuvant suitable for stimulating the host's immune response may be used. A commonly used adjuvant is Freund's complete adjuvant (an emulsion comprising killed and dried microbial cells and available from, for example, Calbiochem Corp., San Diego, or Gibco, Grand Island, NY). Where multiple antigen injections are desired, the subsequent injections may comprise the antigen in combination with an incomplete adjuvant (e.g., cell-free emulsion). Polyclonal antibodies may be isolated from the antibody-producing host by extracting serum containing antibodies to the protein of interest. Monoclonal antibodies may be produced by isolating host cells that produce the desired antibody, fusing these cells with myeloma cells using standard procedures known in the immunology art, and screening for hybrid cells (hybridomas) that react specifically with the target and have the desired binding affinity.

Antibody binding domains also may be produced biosynthetically and the amino acid sequence of the binding domain manipulated to enhance binding affinity with a preferred epitope on the target. Specific antibody methodologies are well understood and described in the literature. A more detailed description of their preparation can be found, for example, in Practical Immunology, (*supra*).

In addition, genetically engineered biosynthetic antibody binding sites, also known in the art as BABS or sFv's, may be used to determine if a sample contains an analyte. Methods for making and using BABS comprising (i) non-covalently associated or disulfide bonded synthetic $V_H$ and $V_L$ dimers, (ii) covalently linked $V_H$-$V_L$ single chain binding sites, (iii) individual $V_H$ or $V_L$ domains, or (iv) single chain antibody binding sites are disclosed, for example, in U.S. Patent Nos.: 5,091,513; 5,132,405; 4,704,692; and 4,946,778. Furthermore, BABS having requisite specificity for the analyte can be derived by phage antibody cloning from combinatorial gene libraries (see, for example, Clackson et al. Nature 352: 624-628 (1991)). Briefly, phages, each expressing on their coat surfaces BABS having immunoglobulin variable regions encoded by variable region gene sequences derived from mice pre-immunized with an isolated analyte, or a fragment thereof, are screened for binding activity against the immobilized analyte. Phages which bind to the immobilized analyte are harvested and the gene encoding the BABS can be sequenced. The resulting nucleic acid sequences encoding the BABS of interest then may be expressed in conventional expression systems to produce the BABS protein.

An isolated analyte also may be used for the development of diagnostic and other tissue evaluating kits and assays to monitor the level of the analytes in a tissue or fluid sample. For example, the kit may include antibodies or other specific binding proteins which bind specifically to one or more analytes and which permit the presence and/or amount of the one or more analytes to be detected and/or quantified in a tissue or fluid sample.

Suitable kits for detecting one or more analytes are contemplated to include, but are not limited to, a receptacle or other means for capturing a sample to be evaluated and a means for detecting the presence and/or amount in the sample of one or more of the analytes described herein. Means for detecting in one embodiment includes, but is not limited to, one or more antibodies specific for these analytes and means for detecting the binding of the antibodies to these analytes by, for example, a standard sandwich immunoassay as described herein. Where the presence of an analyte located within a cell is to be detected (e.g., as from a tissue sample) the kit also may comprise means for disrupting the cell structure so as to expose intracellular components.

The analytes of the present teachings may include nucleic acids of a particular sequence. One or more of the analytes may be detected and/or quantified by determining an amount or absolute concentration of the analyte nucleic acid in a sample, using, for example, Real-Time Quantitative PCR (RT-PCR) and comparing the measured amount to a standard to determine a relative concentration of the analyte nucleic acid in a sample. RT-PCR effectively measures the amount of an analyte nucleic acid resulting from PCR. A positive result represents a measured amount of the analyte nucleic acid that is different than the amount of the analyte from a standard, or a relative concentration having a value above or below zero.

Primers can be developed that are complementary to the nucleic acid sequence of a particular nucleic acid analyte. These primers direct a polymerase to copy and amplify that particular nucleic acid. RT-PCR detects the accumulation of the amplified nucleic acid analyte during the reaction. During the exponential phase of the PCR reaction, the accumulating nucleic acid analyte can be measured. A calibration standard having a known concentration of nucleic acid can be used to prepare a standard curve from which the quantity of the nucleic acid analyte in the tested sample can be extrapolated.

Once the amount or absolute concentration of a nucleic acid analyte in a sample is known, it can be compared to the amount of the analyte from a standard to determine a relative concentration of a nucleic acid analyte in a sample. The standard for classification of major adverse cardiovascular or cerebrovascular event subjects can be determined by empirical means. For example, the amount can be determined by amplifying the nucleic acid analyte in a sample from a population of one or more known normal individuals and quantitatively analyzing the amount of a nucleic acid analyte in the population.

Also, additional forms of chemical analysis of a sample can be performed. For example, quantitative tests can be carried out that indicate the amounts or absolute concentrations of each analyte in a sample. A colorimetric assay is a quantitative chemical analysis measuring color intensity produced by reacting a sample with a reactant as a proxy for the amount of the assayed material in a sample. Reagents can be provided that, when reacted with any analyte, produce a color in the assay sample. The intensity of that color can be dependent on the amount of the analyte in the sample. By comparison of the intensity with a calibrated color card and/or standard, the amount of the analyte in the sample can be determined. This amount can then be compared with the amount of the analyte from a standard (such as from a known normal person) to determine a relative concentration of the analytes in a sample.

Additionally, urinalysis can be used to determine the amount or absolute concentration of an analyte in a urine sample. Urine samples are tested with a variety of different instruments and techniques. Some tests use dipsticks, which are thin strips of plastic that change color in the presence of specific substances. Dipsticks could be used to measure the amount of an analyte.

Moreover, determining a relative concentration by comparing the absolute level or concentration of each of at least two analytes to the level of each of two analytes from a standard can be done as a preventative screening measure and not just when an adverse cardiovascular or cerebrovascular event is observed (i.e., after the disease may have progressed). For example, assuming no evidence of an adverse cardiovascular or cerebrovascular event, subjects could be monitored after a certain age and at predetermined intervals in order to obtain a diagnosis of having or being at risk of having a major adverse cardiovascular or cerebrovascular event at the earliest possible time. To the extent the screen is positive, a medical professional might recommend further monitoring for disease progression, and/or the medical professional might begin treatments with a drug or other therapy.

The results of the analysis, including, for example, the amount or absolute concentration of each of the analytes, the relative concentration of each of the analytes to a standard, and/or a likelihood of having or being at risk of having a major adverse cardiovascular or cerebrovascular event, can be displayed or outputted to a user interface device, a computer readable storage medium, or a local or remote computer system. Displaying or outputting a result or diagnosis means that the results of any of the foregoing analyses are communicated to a user using any medium, such as for example, orally, writing, visual display, etc., computer readable medium or computer system. It will be clear to one skilled in the art that outputting the result is not limited to outputting to a user or a linked external component(s), such as a computer system or computer memory, but may alternatively or additionally be outputted to internal components, such as any computer readable medium. Computer readable media may include, but are not limited to hard drives, floppy disks, CD-ROMs, DVDs, and DATs. Computer readable media does not include carrier waves or other wave forms for data transmission. It will be clear to one skilled in the art that the various sample evaluation and diagnosis methods disclosed and claimed herein, can, but need not be, computer-implemented, and that, for example, the displaying or outputting step can be done by, for example, by communicating to a person orally or in writing (e.g., in handwriting).

Moreover, the analytes can be used to validate animal models of major adverse cardiovascular or cerebrovascular events. For example, in any particular model, a sample could be analyzed to determine if levels of the analytes in the animal are the same as the levels of the analytes in a known major adverse cardiovascular or cerebrovascular event subject. This would validate the model, for example, to test candidate drugs in the manner described above.

Example 1

This example describes the methodology used to identify analytes for major adverse cardiovascular or cerebrovascular events. Briefly, one hundred thrity-six (136) subjects were included in the study. A classification of the subjects into either disease or control categories was achieved by identifying subjects who experienced a major adverse cardiovascular or cerebrovascular event, defined as a myocardial infarction, percutaneous coronary intervention or death, within two years of an index cardiac catheterization.

Frozen plasma samples were obtained from a collection of samples obtained in a long-term study of individuals who had undergone cardiac catheterization at Duke University Medical Center (the CATHGEN Study). The plasma samples were from 136 study subjects, 68 disease cases and 68 matched controls (as defined below). The disease cases and controls were matched according to their coronary artery disease (CAD) index, age, gender and race. Some of the study subjects in both the disease cases and control groups did not exhibit signs of coronary artery disease upon cardiac catheterization and angiography. Other subjects had various degrees of severity of coronary artery stenosis as indicated by higher CAD index scores.

The clinical parameters that were recorded for all samples were those shown in Table 1 below. These parameters, that were not those upon which the disease cases and controls were matched, were included as additional clinical factors in some of the statistical analyses (as discussed below).

Table 1: Clinical Parameters of Study Subjects

| Characteristic | Categories/ Unit | Description |
|---|---|---|
| Age | Years | Age (integer) at Catheterization |
| Race | • Caucasian<br>• African American<br>• Other | |
| CAD Index | | Index of severity of coronary artery disease |
| BMI | • < 25<br>• 25 - 29.9<br>• >= 30 | Body mass index |

(continued)

| Characteristic | Categories/ Unit | Description |
|---|---|---|
| Dyslipidemia | • Yes<br>• No | History of dyslipidemia |
| Family history | • Yes<br>• No | Family history of coronary disease |
| Hypertension | • Yes<br>• No | Yes = History of hypertension or Systolic BP > 140 or Diastolic BP > 90 |
| Diabetes | • Yes<br>• No | Yes = Type I or II diabetes |
| Smoking Status | • Yes<br>• No | History of cigarette smoking |
| Creatinine Clearance Rate | Continuous measure | Cockcroft-Gault approximation based on age, mass, plasma creatinine and gender |
| Daily use of Aspirin | • Yes<br>• No | Obtained from expert curation of medications table |
| Use of Statins | • Yes<br>• No | Obtained from expert curation of medications table |

The disease cases were identified as having the following characteristics. Prior to index catheterization, the subjects had no history of coronary artery bypass graft (CABG) surgery, percutaneous coronary intervention (PCI) for cardiac events or myocardial infoarction (MI). The subjects had no subsequent CABG. The subjects' Ejection Fractions were ≥ 40% (at index catheterization) and not missing. MI, PCI or death occurred within 2 years of index catheterization and blood sampling. Those subjects who clearly had non-coronary artery disease related death (i.e. pulmonary hypertension, cancer, etc.) were excluded. Those subjects with a PCI-related event within 7 days of index catheterization were excluded.

The control cases were identified as having the following characteristics. Prior to index catheterization, the subjects had no history of coronary artery bypass graft (CABG) surgery, percutaneous coronary intervention (PCI) for cardiac events or myocardial infarction (MI). The subjects had no subsequent CABG. The subjects' Ejection Fractions were ≥ 40% (at index catheterization) and not missing. No MI, PCI or death occurred for a period of at least 2 years following index catheterization and blood sampling.

The samples were subjected to comprehensive bioanalysis using the platforms presented in Table 2 below and described in detail below.

Table 2: Bioanalysis Procedures

| Proteomics | Number of Analytes Used in Statistical Analyses |
|---|---|
| LC-MALDI-MS/MS Discovery Proteomics | 217 |
| Targeted Proteomics (Multiplexed Immunoassay) | 66 |
| **Metabolomics** | |
| Lipid LC/MS Metabolomics | 125 |
| Polar LC/MS Metabolomics | 164 |
| GC/MS Metabolomics | 130 |
| Free Fatty Acids | 21 |

A total of 723 analytes were successfully measured in the bioanalysis part of the study. The contribution to that total from each of the bioanalytical platforms is shown in Table 2 above. The bioanalytical platform results were analyzed on a platform-by-platform basis and also combined into a single integrated dataset for subsequent statistical analysis (see discussion below).

Lipid Liquid chromatography ("LC") tandem mass spectrometry ("LC/MS") Metabolomics

This method utilizes liquid chromatography and mass spectrometry conditions that are optimized for resolution and detection of lipid molecules.

The following materials were used according to the method. Solvents and reagents, including water, methanol and isopropanol and Dichloromethane (HPLC grade) were purchased from VWR (USA). Formic acid (99%), ammonium acetate, dichloromethane and reserpine were purchased from Sigma-Aldrich (Milwaukee, WI). HPLC guard column (Symmetry 300 C4 300Å 3.5$\mu$ 2.1x10 mm) and analytical column (C4 300Å 3.5$\mu$ 2.1x150 mm) were purchased from Waters (Milford, Ma). Autosampler vials, 300$\mu$L Total Recovery vials and screw caps were purchased from Waters (Milford, Ma). 500$\mu$L Eppendorf tubes for storage were purchased from VWR. Lipid internal standards 17:0 LPC, 24:0 PC and 40:0 PC were purchased from Avanti Polar Lipids (Alabaster, AL) and 30:0 PC and 51:0 TG were purchased from Sigma-Aldrich (Milwaukee, WI).

The samples were extracted according to the following protocol. Five internal standards were spiked into the extraction solution for data normalization. Stock solutions of internal standards were prepared by dissolving appropriately weighed amounts of each internal standard compound in a 1:9 DCM/IPA solution to achieve final concentrations in the range 1-3mg/ml depending on the particular lipid. The extraction solution was created by aliquoting 300$\mu$L of each of the 5 stock solutions into 300mL of a 1:9 DCM/IPA solution (reference to SOP: BG-QC27 R01). This solution was used as the lipid extraction solution with the five internal standards spiked in with ratios of concentrations of 2:1:2:3:2 ($\mu$g/mL) for 17:0 LPC, 24:0 PC, 30:0 PC, 40:0 PC, 51:0 TG respectively.

The samples were prepared for LC/MS analysis according to the following protocol. Samples of each batch were removed from the freezer together with quality control samples and thawed on ice. An appropriate volume of extraction solution was added to each of the sample vials containing 10$\mu$L of plasma. The vial was vortexed gently and spun down at 14,000 RPM at 24°C for 10 minutes. The supernatant was transferred to an intermediate vial and further mixed by Vortex for a few seconds, 2 aliquots of 60$\mu$L each were pipetted into two bar-coded auto sampler vials for LC/MS analysis as replicates. The remaining supernatant was stored at - 40°C for use in LC/MS/MS analysis. The remaining extract with the pellet was discarded.

The high-performance liquid chromatography (HPLC) analysis was conducted according to the following protocol. Chromatographic apparatus consisted of a Waters 2795 Alliance HPLC system with a quaternary gradient system, auto sampler, an external column heater set to 45°C and an analytical HPLC column with an in-line pre-column. Analytes were separated and delivered to the mass spectrometer at a flow rate of 350$\mu$L/min. A binary gradient (0 min- 20%B, 2 min- 20%B, 4 min- 80%B, 20 min- 100%B, 25 min- 100%B, 25.1 min- 20%B, 35 min- 20%B) was used for analyte separation with solvent A comprising of 95% $H_2O$ and 5% MeOH and solvent B constituting 99% MeOH and 1% $H_2O$. Both solvent streams contained 10 mM ammonium acetate and 0.1% formic acid. 5$\mu$L of sample was injected into a 20$\mu$L sample loop and loaded onto the precolumn prior to separation by the analytical column. Between each sample, the injection needle, loop, and syringe were washed with isopropanol/MeOH solution

LC/MS analysis was performed on a Waters/Micromass quadrupole time-of-flight instrument (Waters, Q-ToF™) equipped with a LockSpray ESI source. The ESI source block temperature was maintained at 120°C and the desolvation temperature was set at 320°C. MS signal sensitivity was optimized using the reserpine peak at 609.2814. Mass calibration was performed in MS mode using protonated peaks of polyalanine. Average instrument resolution of ~8500 was targeted for the 5 IS Peaks with a LockMass Calibration accuracy of 3 to 5 ppm. On the basis of previous studies, MS data was acquired from 7 to 23 minutes during LC elution. All LC/MS data were acquired in centroid mode by scanning m/z 300-1000 in 0.3 seconds.

Subsequent MS/MS analysis for analyte identification was performed on the same LC/MS system with the identical LC condition. MS/MS calibration was performed using fragments of polyalanine peaks of precursor at m/z 1084. All MS/MS data was acquired in centroid mode by scanning *m/z* 100-1000 in 1 or 2 seconds. When necessary, LC retention time correction was applied to the target list. MS/MS data is collected by targeted analysis.

Peak detection was conducted according to the following protocol. The raw data files of the accepted runs were peak picked and integrated. Only peaks reaching the defined thresholds were advanced into the alignment process. Each peak was characterized by *m/z* value and retention time, for example, a peak detected at *m/z* 510 and eluted at 7.88 minutes would be labeled as 510_0788. Each sample is processed independently.

After the peak-picking is completed, the .MLL files generated by the peak-picking algorithm were "aligned" to each other. The program first sets a retention time window using the Internal Standard peaks from all injections and then logically "aligns" each analyte within the batch based on the mean retention time of the Reference peaks. At the end of the process, the physical retention time may be changed from 510_0785 to 510_0788 and an additional threshold may be applied to the peak areas.

Quality control (QC) was assessed for each batch by many measurements, including calculating the percent relative standard deviation (% RSD) of the internal standards across all samples, plotting the extracted areas (intensity) of internal standard peaks as a function of time to identify any trends and checking the retention time variations both pre and post alignment. Visual QC inspection of each injection also took place during the analyses. These reviews focused on how well the replicate injections overlay, the behavior of the QC samples over time and general instrument trends. Minor changes in retention and peak area that may arise during the study were corrected in the data normalization process (slight temporal trends for example) but more significant changes (like partial injections) may have required reanalysis.

In the measurement process of LC/MS, each molecular species of interest typically appears as multiple chemical entities. This can be due to several factors: first, the presence of multiple stable isotopes of constituent elements, and second, the formation of pseudo molecular ions of the analytes via adduct formation with sodium (Na), potassium (K), ammonium ($NH_4$), and other analytes resulting in multiple types of ions. In addition, the mass spectrometry ionization process can be often accompanied by fragmentation of the original molecule. Consequently, acquired data-sets are inflated by an order of magnitude in the number of peaks that represent an analyte. These additional peaks which are redundant in their information content, contribute to the introduction of noise, decrease of statistical power, masking of effects in bioinformatics analysis, and because of the large number of peaks, may make it more difficult to draw clear scientific conclusions. An automated computational platform, which groups measured entities according to their putative compounds of origin, was used following peak-picking and alignment of spectra, and prior or parallel to data normalization and statistical analysis. The core algorithm scans the data according to retention time (RT) with a narrow moving time window. Candidate peaks of similar retention time are grouped according to known mass difference patterns depending on the specific chemical characteristics of the platform.

Following the peak detection and alignment, several additional steps of data processing occur prior to the data being available for univariate and multivariate statistical analyses. These steps include removal of peaks that are assigned to sample/study blanks and other background peaks, batch correction and normalization. The completion of these steps represents the milestone referred to as "Locked Data Set".

Mass Spectrometry profiling and the ensuing data processing generated a list of analytes for subsequent statistical analysis. The primary analytes, usually the most abundant peak for each analyte family, were subjected to targeted MS/MS using the same or similar LC/MS set up as the initial profiling experiment. Targeted LC/MS/MS spectra were acquired in centroid mode. MS/MS spectra obtained by targeted MS/MS analysis of the analytes were interpreted by a combination of retention time, mass, Nitrogen-rule, fatty acid (FA) side-chain specific fragment ions, and comparison with reference spectra from a library of MS/MS of lipids.

Gas chromatography tandem mass spectrometry (GC/MS) Metabolomics

GC has the advantage of high separation efficiency and robust retention times combined with sensitive and selective (electron impact) mass detection. However, many compounds contain polar functional groups that are either thermally labile at the temperatures required for their separation or are not volatile at all. In addition, the peak shape of compounds with polar functional groups can be unsatisfactory because of undesired column interaction such as irreversible adsorption. In order to address these issues, derivatization of the compounds prior to GC analysis can be necessary.

The following materials were used according to the method. Solvents and reagents included methanol; pyridine; ethoxyamine hydrochloride; and N-methyl-N-trimethylsilyl trifluoroacetamide (MSTFA). Internal standards included leucine-D3; phenylalanine-D5; glutamic acid-D3; glucose-D7; cholic acid-D4; alanine-D4; trifluoroacetylanthracene (TFAA); difluorobiphenyl (DFBP); and dicyclohexylphthalate (DCHP).

The samples were thawed at room temperature and 10 μL of a 250 ng/μL standard of leucine-D3, phenylalanine-D5, glutamic acid-D3 and glucose-D7 in H2O was added followed by 400 μL of methanol (for protein precipitation). The samples were vortexed, centrifuged for 10 minutes at 10000 rpm and the supernatant transferred into an autosampler vial. After a drying step with nitrogen, 10 μL of a 250 ng/μL standard of cholic acid D4 and alanine-D4 in pyridine was added. Before capping the autosampler vial, 30 μL ethoxyamine hydrochloride solution in pyridine was added and the sample was incubated at 40°C for 90 minutes.

After the oximation, the remaining internal standards were added: 10 μL of a 250 ng/μL standard of difluorobiphenyl, dicyclohexylphtalate and trifluoroacetylanthracene in pyridine. The sample was silylated by adding 100 μL MSTFA and heating for 50 minutes at 40°C. Before injection in the GC, the samples were centrifuged for 10 minutes at 3500 rpm.

GC/MS analysis employed an Agilent 6890 N gas chromatograph equipped with a PTV (programmed temperature vaporizer) injector and a CTC Analytics Combi-Pal autosampler. For detection, an Agilent 5973 Mass Selective Detector was used. The system was controlled by Enhanced Chemstation G1701CA Version D.01.02 software.

All compounds eluting from the GC/MS column were detected in full scan mode. Each peak was characterized by its retention time and a number of fragments (m/z values). The amount of data (i.e. number of variables) was reduced by applying a target processing procedure.

The target approach reduces the number of variables by at least a factor of 20. Most of the procedure artifacts could be removed from the data by not including these peaks in the target table. A study specific target table was compiled for this study. Information from previous human plasma GC/MS projects were used in this process. A number of representative chromatograms were selected from the study samples, including extreme samples to include as many peaks observed in this study as possible in the target table. During the initial stage of the project the identity of the peaks was not known and they were labeled 'unknown 1' to 'unknown x', and each peak was characterized by its retention time and one quantifying ion. Peaks found in the procedure blanks were removed from the target table. In cases of the uncertainty of origin of a peak, the peaks were retained in the target list to avoid potential loss of relevant analytes.

In study samples, the individual analyte/compounds, other than internal standards, found in the representative chromatograms were matched with the reference compound database (retention times and mass spectra). After this matching step, a number of compounds were assigned a tentative ID based on the reference database.

Retention time alignment of peaks is not required when a targeted processing approach is applied. The target table can be adjusted in cases where retention time shifts were observed (the internal standards served as 'landmarks'). All compounds present in the target table were first integrated using standard integration settings. These settings are not applicable to all compounds and samples and may lead to incorrect integration results for some compounds. A procedure has been developed to find integration errors. For all targets, the deviation of the peak area from the mean (of all study and QC samples) can be calculated. The results were plotted and used to detect problem peaks (integration errors for those peaks spanning multiple samples) and problem samples (integration for multiple peaks within a sample). For the problem samples, the integration was performed manually. For problem peaks automated integration was performed after adjusting the integration settings).

At various steps in the sample preparation and analysis, one or more internal standards were added in order to monitor the quality of the data after analysis. After each batch, the response of the internal standards after correction for DCHP was evaluated in all samples. If the corrected peak area of each internal standard deviated less than 20% from the batch mean the batch can be approved. If, for one or more samples, the deviation is more than 20% then those samples were reanalysed in the next batch. Ultimately a Locked Data Set can be generated for the GC/MS analysis.

The first identification step can be matching of the target compound list with the reference standard database. A number of compounds from the target list may be identified and confirmed by comparison with the reference databases and analysing reference standards. Following univariate and multivariate analysis, the prioritized unknowns were first evaluated by checking the raw data and the QC results. They were divided into two categories: i) compounds with very low intensity and/or high RSDs in the QC standards and ii) compounds with good signal in one or more samples and low RSDs in the QC standards. Identification starts with the second category. For both categories, additional check for artefacts were performed.

After this analysis, additional identification methods were selected depending on the individual (to be identified) compounds. For some compounds, hits were found in commercial spectral libraries and no additional ID experiments were required. For other compounds chemical ionization, accurate mass or other derivatization experiments were performed.

Polar LC/MS Metabolomics

The following materials were used according to the method: Methanol, Biosolve, LC-MS grade, cat.# 13687801, or equivalent; HCl, Merck, 37% cat.# 1.00317, or equivalent; n-butanol, Baker cat.# 8017, or equivalent; Milli-Q water, water purified with ELGA System, or equivalent; Dithiothreitol DTT, Sigma 99%, cat.# D9779, or equivalent; Formic acid, Merck cat.# 1.00264, or equivalent; Acetonitrile, Biosolve HPLC-S Gradient grade, cat.# 12000701, or equivalent; Phenylalanine-d5, CDN Isotopes 99 atom % D, cat.# D-1597, or equivalent; Glutamate-d3, CDN Isotopes 99 atom % D, cat.# D-1196, or equivalent; Leucine-d3, CDN Isotopes 99 atom % D, cat.# D-1973, or equivalent; Alanine-d3, CDN Isotopes 99 atom % D, cat.# D-1462, or equivalent; Creatine-d3, CDN Isotopes 99 atom % D, cat.# D-1462, or equivalent; Methionine-d4, CDN Isotopes 99 atom % D, cat.# D-1462, or equivalent; Methyl-(d3)-histdine, CDN Isotopes 99 atom % D, cat.# D-1462, or equivalent; Tyrosine-d7, CDN Isotopes 99 atom % D, cat.# D-1462, or equivalent.

The following instruments were used according to the method: Vortex; Centrifuge; Oven; Evaporation equipment; ThermoFinnigan Surveyor or Surveyor Plus HPLC; ThermoFinnigan LTQ ion trap mass spectrometer equipped with ESI source.

To a 10μl plasma sample in a small Eppendorf vial, 10μl IS-work solution was added and the sample was vortexed briefly. After addition of DTT solution and deproteinization of the sample, the supernatant was lyophilized. The samples were then derivatized with HCl-butanol at 65°C. The excess of the reagent was removed by lyophilization. The sample was reconstituted in a water solution of DTT containing underivatised Tyrosine D7 as the internal standard.

High performance liquid chromatography was performed according to the following specifications:

Column:                                          Varian/Chrompack Inertsil 5μm ODS-3 100*3mm

(continued)

| | |
|---|---|
| Guard column: | Varian/Chrompack R2 10 x 2 mm i.d. |
| Mobile phase A: | 0.1% formic acid |
| 1 ml formic acid is added to 1000 ml water, mixed and degassed by ultra-sonication for 5 minutes | |
| Mobile phase B: | 80% Acetonitrile in 0.1% formic acid |
| 800 ml Acetonitrile, water ad 1000 ml and 1 ml formic acid mix and degas by ultra-sonication for 5 minutes. | |
| Column temperature: | 25°C-30°C |
| Autosampler temperature: | 10 °C |
| Injection volume: | 10μl |

Mass spectrometry was conducted with a split with a ratio of approximately 4:1 which means a flow of ~ 75 μl to the ESI source. The ESI and MS settings are:

| | |
|---|---|
| ESI spray voltage: | 4 kV |
| heated capillary: | 250-300°C |
| sheath gas: | 50-60 |
| aux gas: | 5 |
| polarity: | positive |
| scan range: | 125-1250 |
| number of microscans: | 5-6 |
| max injection time: | 200 ms |
| source CID: | 0-5 V |
| source distance | position C |

Selected samples were analysed for accurate mass. For this purpose, two QC samples from each derivatisation lot were selected and also a number of "extreme" samples. Accurate mass experiments were performed on a ThermoFinnigan LTQ-FTMS instrument, using the same settings as described above. The detection of the ions was performed in the FTMS. Resolution at m/z 400 is ~100,000.

All compounds eluting from the LC/MS column were detected in full scan mode. Each peak was characterized by its retention time and a number of ions (m/z values). The amount of data (i.e. number of variables) was reduced by applying a target processing procedure, where each compound in the chromatogram was in most cases represented by only one entry in the target table.

The target approach reduced the number of variables by at least a factor of 20. Most of the procedure artifacts could be removed from the data by not including these peaks in the target table.

A study specific target table was compiled for this study. This was performed by peak picking of all QCY and study samples from the first few batches. The resulting peak table was sorted based on intensity and the 1000 highest peaks were selected. From this partial table, isotope peaks and as far as recognized, adducts were removed. At this stage, the identity of the peaks was not known and they were labeled as m/z at retention time (in minutes).

Retention time alignment of peaks is not required when a targeted processing approach is applied. The target table was adjusted in cases where retention time shifts were observed (the internal standards served as 'landmarks').

After completion of the analysis of all runs, all compounds present in the target table were first integrated using standard integration settings. These settings were not applicable to all compounds and samples and can lead to incorrect integration results for some compounds. A procedure was developed to find integration errors. For all targets, the deviation of the peak area from the mean (of all study and QC samples) was calculated.

The quality control of the complete dataset was performed based on the IS response in all the samples except blanks and the check of relative standard deviations of all the target compounds in the QCY and CTRL samples. These results were compared with those observed in previous studies. At the completion of these steps, a "Locked Data Set" can be generated for the Polar LC/MS Platform. This data set forms the foundation for all subsequent statistical analyses.

The first identification step was matching of the target compound list with the reference standard database containing a number of amino acids and related compounds. Retention times and accurate masses were compared and, where necessary, MS/MS spectra were compared.

The remaining target compounds were identified only after they were listed on the priority lists (univariate/multivariate). The prioritized unknowns were first evaluated by checking the raw data and the QC results. They were divided into two categories: i) compounds with very low intensity and/or high RSDs in the QC standards and ii) compounds with good signal in one or more samples and low RSDs in the QC standards. Identification started with the second category. For both categories, additional check for artefacts was performed; the blank analysis was checked for the presence of these compounds.

For further identification, retention time, accurate mass and MS/MS data were used. Based on accurate mass and the knowledge about the derivatization used, possible elemental compositions were searched for in KEGG, Merck and Chemfinder databases. The possible hits were evaluated and for this purpose, the retention times and the MS/MS spectra were used. In several cases, standards were purchased and analysed in solution and after standard addition to the study samples.

LC/MS Profiling of Free Fatty Acids

Plasma was deproteinized and extracted with IPA. Free fatty acids were separated on an analytical column, ionized by electrospray in negative ion mode and detected in full scan mode. A number of free fatty acids were analyzed as external standards and if real concentrations in plasma needed to be determined, these were calculated (approximated) using the calibration standards.

Samples were prepared and analysed using the following materials. Instruments included a centrifuge for eppendorf-tubes, a Thermo Electron LTQ, Thermo Electron Surveyor HPLC with autosampler and column-oven, and Vortex. Chemicals included Demineralised water, (ELGA System 4 or any other make, e.g. Millipore); Isopropanol p.a., (Baker 6068 or equal); Methanol, (HPLC grade, Chromasolve 34966); Dichloromethane p.a., (Baker 7053 or equal); Ammonium acetate p.a., (Fluka 09690 or equal); and Formic acid p.a., (Merck 1.00264.1000 or equal).

Material included Alltech Prosphere C4 300Å HPLC column (150 x 3.0 mm i.d., 5 $\mu$m), partno. 35548; Symmetry 300 C4 guard column (10x 2.1 mm i.d. 3.5 $\mu$m), partno. 186000278; Autosampler vials (Alltech partno. AV055201, 32*11.6 mm); Eppendorf tubes; and Pipettes (e.g. Eppendorf).

The following standards were used: Heptadecanoic acid C17:0 FA (Sigma-Aldrich, H3500); C14:0 FA Myristic acid (Sigma M3128); C16:0 FA Palmitic acid (Sigma P5585); C16:1 FA Palmitoleic acid (Sigma P9417); C18:0 FA Stearic acid (Sigma S4751); C18:1 FA Oleic acid (Sigma 01008); C18:2 FA Linoleic acid (Sigma L1376); and C20:4 FA Arachidonic acid (Sigma A9673).

Internal standard solutions were prepared according to the following methods. Stock solutions of Heptadecanoic acid C17:0 FA (1 mg/mL) were prepared by weighing 5 mg in a 5 mL volumetric flask, adding 500 $\mu$L DCM, dissolving by ultrasonicating and filling up to 5 mL with IPA. The IS working solution (for protein precipitation & extraction of lipids) was prepared in IPA containing the C17:0 FA in a concentration of 1 $\mu$g/mL.

Samples were prepared according to the following method. 300 $\mu$L of IS working solution was added to 10 $\mu$L of thawed plasma sample in an eppendorf cup and vortexed. The solution was subjected to eppendorf centrifuge for 3-5 minutes at 10,000 rpm and transfered 2 times of 50 $\mu$L of the the clear supernatant into two autosampler vials with an insert and the remaining extract was stored at <-18°C.

Liquid chromatography and mass spectrometry was performed using an Alltech Prosphere C4 300Å HPLC column (150 x 3.2 mm i.d., 5 $\mu$m) and a Phenomenex Widepore C4 guard column (4 x 3 mm i.d., 5 $\mu$m). The mobile phase A consisted of 5% Methanol in buffer; the mobile phase B consisted of Methanol, containing 2 mM $NH_4Ac$; and the needle wash consisted of 60% IPA in MeOH. The gradient was as follows:

| Time (min) | Flow (mL/min) | %A | %B |
| --- | --- | --- | --- |
| 0 | 0.4 | 70 | 30 |
| 2 | 0.4 | 70 | 30 |
| 6 | 0.4 | 30 | 70 |
| 10 | 0.4 | 0 | 100 |
| 15 | 0.4 | 0 | 100 |
| 15.1 | 0.4 | 70 | 30 |
| 20 | 0.4 | 70 | 30 |

The column temperature was 40 °C; the autosampler temperature was 20 °C; and the injection volume was 20 $\mu$L.

For the mass spectrometry procedure, the LC/MS instrument was tuned using the following calibration standards:

| Instrument | ThermoFinnigan LTQ |
|---|---|
| Mode | negative |
| Heater capillary | 250°C |
| Spray voltage | 3.5 kV |
| Sheath gas | 35 arb |
| Auxilary gas | 15 arb |
| Spray position | C |
| Scan range | 180-400 |
| Scan speed | Auto |
| Multiplier | Auto |

Thermo XCalibur LCQuan V 2.0 was used for the integration of selected target analytes. Data was processed using a target table, containing at least the following fatty acids (based on previous studies): C12:0 FA, C12:1 FA, C14:0 FA, C14:1 FA, C16:0 FA, C16:1 FA, C16:2 FA, C18:0 FA, C18:1 FA, C18:2 FA, C18:3 FA, C20:0 FA, C20:1 FA, C20:2 FA, C20:3 FA, C20:4 FA, C20:5.FA, C22:5 FA, C22:6 FA, and C22:7 FA.

00] Integration data for all the compounds from the target table was obtained. The result was an Excel table containing an integration value for each target compound.

LC-MALDI-MS/MS Discovery Proteomics

The proteomics workflow was based on a multi-dimensional liquid chromatography - MS/MS analysis of peptides generated from the plasma samples. Advantages of this approach include the insensitivity to multiple heterogeneities of plasma proteins that cause difficulties in a 2D gel based approach, ready automation of the liquid chromatography techniques, and the possibility to utilize stable isotope labeling of peptides.

The recently introduced iTRAQ™ methodology (iTRAQ is a trademark of Applied Biosystems) was particularly suitable for this study as the labeling reaction was easy to carry out to completion, generic, almost all the peptides get iTRAQ labeled, and it allowed for multiplexed quantification of up to four samples per experiment. The latter was important in this study where consistent quantification (and identification) of the same proteins from sample to sample is a key requirement. Another significant advantage of stable isotope labeling was the robustness of the process with respect to variations of liquid chromatographic behavior of peptides from sample to sample (once differentially labeled pools were combined).

In the iTRAQ method, enzymatic digests from protein samples are treated with the reagents (an N-hydroxysuccinimide (NHS) ester) that derivatize the free primary amino groups of peptides: the N-terminus (if not blocked) and the lysine residues. Four different varieties of the reagent are characterized by the same mass but different positioning of the stable isotope labels. Therefore, in MS mode, where the ion signal reflects the molecular weight of the peptides, all four differently labeled peptides appear as a single component. In MS/MS mode, the subtle differences in the structure of the label become visible and, whereas the peptide backbone fragments (so-called a, b, and y) are still isobaric, four different reporter fragments are generated corresponding to m/z 114, 115, 116, and 117, respectively. Relative quantification of the peptides from four different samples can be accomplished through the determination of relative intensities of these reporter ions.

In this study, a MALDI TOF/TOF mass spectrometer (ABI 4800) was used in which the samples were deposited onto MALDI plates through an automated fraction collection procedure. The particular advantage of MALDI LC-MS/MS is in the off-line nature of the LC-MS coupling. It was possible to analyze an entire 2D LC separation in MS mode first, before generating peptide identifications and quantitative data in MS/MS mode. This feature can be very convenient when a constant set of peptides have to be measured throughout a large number of samples.

One unique aspect of analyzing plasma samples is the enormous background represented by the abundant plasma proteins (albumin, immunoglobulins, etc.). To address this challenge, a number of depletion techniques were used. In this study, a chicken IgY antibody column was used to deplete the samples of twelve abundant proteins. The protein pool not retained on the antibody column was recovered on a reversed-phase column. The recovered protein pool was reduced, alkylated and digested by trypsin. The resulting peptide mixture was labeled with the iTRAQ reagent and combined with the three other samples designated for the same iTRAQ mix. The combined four-plex mixes were fractionated first on strong cation exchange chromatography. Ten fractions were collected from the SCX elution. These fractions were analyzed further - after pooling some of the fractions - through HPLC MS/MS.

iTRAQ analysis allows for the relative quantification of four different samples in a single 2D LC-MS/MS experiment (four-plex). However, assigning four primary samples into an iTRAQ four-plex mix does not allow comparing two primary samples designated to different iTRAQ mixes. This was addressed by creating a reference pool that was aliquoted according to the number of iTRAQ mixes analyzed for the project; a constant component of each iTRAQ mix; sample preparation for this reference aliquot was carried out along with the three other members of the same iTRAQ mix; and, labeled with the 117 reagent.

The reference pool was created from a combination of approximately 25% of each primary study sample analyzed in the study. The aliquots from the reference pool were referred to as QCR samples.

MS/MS acquisition can be guided by lists of MS precursors for both inclusion and exclusion. An original inclusion list was derived from the analysis performed during the catalog phase of the study. In this phase, a few samples from the QC Pool of the samples were processed via the complete proteomics workflow. After identifying peptides and matching proteins from these mixes the inclusion and exclusion lists were compiled.

Content of the Inclusion list includes: fully alklyated and iTRAQ labeled tryptic peptides; fully alklyated and iTRAQ labeled semi-tryptic peptides with the exclusion of those due to chymotryptic activity (cleavage at F, Y and W); and peptides manually identified as having post-translational modifications not routinely search for by Mascot.

Content of the Exclusion list includes: 2,000 most abundant unidentified components, including peptides with incomplete iTRAQ labeling or alkylation; peptides in excess of 3,000 Da molecular weight containing multiple missed tryptic cleavage sites; peptides generated by chymotryptic side-activity of trypsin; potential M+K$^+$ form of abundant peptides that were on the inclusion list in M+H$^+$ form; and autolytic peptides from trypsin.

A custom software application was used to consider inclusion/exclusion criteria and spawn MS/MS acquisitions on the AB4800 based on separate text files itemizing precursors selected for either inclusion or exclusion. Automatic adjustments of potential shifts of the HPLC retention times were also accommodated by the software. Additional MS/MS spectra were acquired in an opportunistic manner as acquisition time permitted after first analyzing included precursors.

Samples were thawed on ice in daily batches of 12 consisting of 9 primary samples and 3 reference pool samples and were serially processed with one reference pool sample following three primary samples (i.e. samples 1, 2, 3, then R, 4, 5, 6, R etc.). Once the samples were thawed completely, they were delipidated. Samples were diluted with PBS and extracted with trichlorotrifluoroethane (Freon 13) followed by a centrifugation step. The top aqueous phase was transferred to a new tube and stored briefly on ice before being placed in the chilled auto-sampler of the LC system for abundant protein removal.

Using Abundant Protein Removal (APR), delipidated samples were depleted of twelve of the most abundant plasma proteins. This procedure used a two-column method (2DLC) where twelve proteins including albumin, IgG, IgA, transferrin, α-1 antitrypsin, the haptoglobin family, fibrinogen, α-1 acid glycoprotein, IgM, α-2 macroglobulin, and HDL (Apolipoproteins A-I and AII) were targeted by an affinity IgY column while depleted material flowed through and were captured on a reversed-phase (RP)column. The RP column was independently washed and step eluted. The elution peak, consisting of the washed, depleted material, was collected in a fraction collector and later dried down by speed vacuum for further processing in the workflow. The RP column was then washed with a stripping solution that eliminates carry-over to the next run. The IgY column was then independently eluted and neutralized before the next run. Twelve delipidated samples (a single batch) were processed in a 24-hour period followed by an overnight speed vacuum of the collected fractions.

Depleted plasma samples were reduced, alkylated, digested, and iTRAQ labeled. Dried down depleted samples were re-suspended in 1M TEAB Buffer pH 8.5. TCEP and SDS were added to denature and reduce the samples. After an hour-long incubation at 71°C the samples were alkylated with iodoacetamide for 30 minutes at room temperature in the dark. Trypsin suspended in N-acetyl-L-cysteine was added and the samples were allowed to digest overnight at 48°C. The next day, iTRAQ reagents were thawed and re-suspended in 100% ethanol. Samples were labeled with specific iTRAQ reagents. After an hour of incubation at room temperature for the labeling, the acetylation reaction was quenched with the addition of 4 microliters of 1M ammonium-bicarbonate. The iTRAQ labeled samples for the same mix were combined and dried down in a Speedvac before re-suspension for cation exchange chromatography.

Strong cation exchange fractionation of the combined iTRAQ mixes was carried out on a Vision Chromatography Station (Applied Biosystems) using a 4.6 x 50 mm Poly- Sulfoethyl Strong Cation Exchange Column. The separation used a two-column method where the peptide pools are loaded to a Poros R2 reversed phase column first in order to remove the large amount of iTRAQ hydrolysis products.

The peptides were eluted onto the SCX column by using an SCX loading buffer of 35% ACN content. More hydrophobic material stuck on the RP column (i.e. residual trypsin) was removed by high-organic wash. The peptides bound on the SCX column were eluted with a gradient. Prior to injection, the pH of the sample is adjusted to 3.5 using 1N HCl. From the SCX separation, ten fractions of 0.8 mL (A1 through A10) were collected. These fractions were dried down in a Speedvac and re-suspended in HPLC loading buffer.

LC-MALDI-MS/MS Discovery Proteomics

The coupling of HPLC and MALDI was carried out off-line, through fraction collection onto the MALDI target. The equipment was an UltiMate Chromatography System equipped with Probot MALDI spotting device from Dionex-LC Packings (Hercules, CA). An integrated syringe pump on the fraction collector enabled the co-mixing of HPLC eluent and MALDI matrix prior to deposition onto the MALDI plate. To ensure good mass calibration of the MALDI MS spectra, an internal standard - ACTH (18-39) peptide; MH+=2465.199 - was added to the matrix solution.

Spotting to the MALDI plates was configured in a way that three HPLC runs were deposited onto a single 4800 MALDI plate. Each HPLC runs contained a 6x35 array of fractions (210 fractions/HPLC). The six HPLC runs representing an iTRAQ mix were spotted onto two MALDI plates.

The HPLC-MALDI operating parameters are listed below. HPLC grade Water and Analytical Grade Acetonitrile were from Burdick & Jackson, ammonium acetate, ammonium citrate, TFA, and $\alpha$-cyano-4-hydroxycinnamic acid were from Sigma.

HPLC MALDI parameters:

| | |
|---|---|
| HPLC Buffer A: | 95% water, 5% acetonitrile, 0.1% TFA |
| HPLC Buffer B: | 10% water, 90% acetonitrile, 0.1% TFA |
| HPLC loading buffer: | 95% water, 5% Acetonitrile, 0.1% TFA, 2mM ammonium acetate |
| Matrix solution: | 10 g/L $\alpha$-cyano-4-hydroxycinnamic acid - 0.1 g/L dibasic ammonium citrate in 15% water, 85% acetonitrile |
| Internal mass ACTH (18-39) peptide at 200 fmole/ L (in matrix calibration standard: solution) | |
| HPLC column: | resolving 0.180x150 mm C 18 (Dionex-LC Packings) |
| HPLC trap column: | 0.3x5 mm C18 (Dionex-LC Packings) |
| HPLC flow rate: | 2 microL/min |
| matrix flow rate: | 2 microL/min |
| fraction collection interval | 9 sec |

The HPLC gradient was programmed as:

| | |
|---|---|
| 0 - 5% B | in 8 min. |
| 5-10%B | in 2 min. |
| 10 - 37.5% B | in 30 min. |
| 37.5 - 100% | B in 2 min. |

UV traces were recorded using a 45-nL flow cell monitoring the wavelength of 280 nm.

The injected amounts of each SCX fraction were determined to maximize the sample load but avoiding overloading the HPLC column. These amounts were determined in preliminary catalog experiments.

Prior to each set of six injections representing the MALDI samples from an iTRAQ mix, a standard mixture of peptides (from Michrom) was injected to monitor the consistency of HPLC retention times. Note that moderate changes in HPLC elution times were seamlessly handled by the precursor selection program by automatically aligning the LC-MS runs of adjacent SCX fractions. Following the standard QC mix, the six SCX fractions were injected and two MALDI plates were generated.

Sets of two MALDI plates were mounted in the autoloader of the AB4800 instrument. Each plate was run in MS mode through the following sequence:

- Generate plate model & update default calibration using the 4800 standard mix (Applied Biosystems) spotted on the eight "Cal" positions on the plate
- Run the LC fractions in MS mode with internal mass calibration using the *mlz* 2465.199 (peptide std.) and *mlz* 568.136 (matrix trimer) peaks

The major objectives for precursor selection were aimed at minimizing the acquisition times (eliminate redundant precursors), eliminating interference from adjacent peaks in the MS spectrum and consistently selecting the same precursors for all the iTRAQ mixes. The MS/MS acquisition and processing parameters were optimized to produce the best possible measurement of the iTRAQ reporter peaks at m/z 114, 115, 116, 117. Note that the MS/MS acquisitions were set to accumulate 2,100 laser shots unless the iTRAQ signal exceeded the signal-to-noise criteria after only 1,050 shots. In this way the acquisition time could be reduced without risking the quality of iTRAQ peak area measurements.

AB4800 MS/MS spectra were harvested from the Oracle database resident on the acquisition control computer using a custom Perl script driven by pipeline control software. MS/MS peaks were processed into two distinct files to separately record the quantitative iTRAQ peak data and the peptide fragmentation peaks. Each individual MS/MS spectrum was identified by a unique database peak table ID and the unique database ID of the MALDI plate from which the data was acquired. These two values permitted further association with additional information recorded in both the acquisition data and the internal Laboratory Information Management System (LIMS). The iTRAQ quantitative information was transferred into a database called PeptidedB from the first file. The secondary output was an MGF (Mascot Generic Format) file containing the peptide fragmentation peaks and additional associated comment lines to permit tracking between Mascot results and input MS/MS spectra.

The peaks included in the MGF files were filtered to remove high mass peaks (within 64 Da or the precursor $MH^+$); remove low mass peaks (< 146 Da); and limit the "density" of peaks to 15 fragment masses per 200 Da window.

A separate MGF file was created per MS/MS job run on the AB4800 (an MS/MS job can consist of between 1 and thousands of separate MS/MS acquisitions acquired from the same MALDI plate). Individual MGF files corresponding to the same iTRAQ mix were concatenated prior to initiating a Mascot search.

Each MS/MS spectrum was associated with the ion intensity of the *mlz* 114, 115, 116, and 117 ion signal in PeptideDB. As described above, iTRAQ ratios were determined relative to the *mlz* 117 intensity (since this label was used for the reference pool). Ratios were computed as: ratio of peak areas for 4800 data (not cluster areas as used by Applied BioSystems software) and ratio of peak counts for QToF data.

Following database searching, peptide sequences matched to MS/MS spectra "inherited" the quantification numbers assigned to the MS/MS spectra. Protein ratios were derived from the ratios of the peptides assigned to that protein as a mean ratio (or average ratio in the Applied Biosystems software). However, the final peptide-protein assignments in the BGM workflow were not made until peptide identification was completed from each iTRAQ mix. At this point, not just proteins assignments were consolidated, but individual proteins may be broken up into multiple nodes based on the quantitative information for the entire study (*i.e.* protein isoforms, peptide sequences accommodating polymorphic sites, allelic variants, *etc.* would be resolved). Protein nodes - that are ultimately used for statistical and Correlation Network analysis - were then represented as the mean of the peptide ratios matching to them.

When propagating peptide ratios into protein or protein node ratios, only those peptides were considered that were (a) fully modified to the expected extent; *i.e.* fully iTRAQ labeled and fully alkylated if they contain cysteine(s) or (b) not matching to proteins targeted by APR, or to trypsin, to hemoglobin (these proteins are considered contaminants)

Peptide/protein identification was carried out using a three-pronged approach of the commercial Mascot database search program (MatrixScience Ltd., UK), expert validation by scientists, and an MS/MS spectral matching procedure.

Once the data acquisition was complete for the entire proteomics component of the study and the Mascot search results are loaded into PeptideDB, a spectral matching procedure was run. The objective was to find those MS/MS spectra that match to a peptide sequence to the criteria of auto-validation in *any* of the iTRAQ mixes analyzed in the proteomics project. Peptide identity was established based on closely matched: precursor mass; SCX fraction number and HPLC retention time; and masses of most abundant fragment ions. This way it was possible to rescue a number of MS/MS spectra that did not meet auto-validation criteria by Mascot but had an auto- or expert validated instance in other iTRAQ mix(es). The importance of such procedure is to increase the representation of peptides and proteins measured across as many samples in the proteomics study as possible.

After peptide identification was complete for the entire study (having the most complete peptide set possible), a protein validation tool was run to identify the minimum set of proteins explaining all the validated peptide sequences. In the terminology of the protein validation tool peptide, sequences were assigned to (a) protein class standing for a unique gene in the genome or (b) protein exemplar standing for the preferred (usually the one having the most annotation information) sequences instance in a protein class (amongst splice variants, polymorphic forms, *etc.*).

A number of data processing steps were applied to the complete data set and influenced the number of proteins/peptides and quantifications metrics reported for the study. These data processing steps include:

*Consolidation of peptide measurements:* iTRAQ signals from mutliple MS/MS measurements of the same peptide (same sequence and same modifications) in the same iTRAQ mix were summed and a single ratio measurement used from that point on.

*Outlier elimination:* A standard statistical procedure to remove 5% of outlier peptide measurements. A peptide measurement was considered outlier if it fell well outside (more than two standard deviations) the distribution of all peptide measurements matching to the same protein.

*Final protein/node assignment:* Once the protein validation tool analysis was complete, quantitative results for the entire project were used to finalize the protein list for univariate and multivariate statistical analysis. Final proteins defined the nodes for correlation network analysis. This process was necessary for the correct tracking of processed forms of proteins (i.e., C3a and C3b from Complement C3, Fibrinopeptide A from fibrinogen alpha chain, alpha-microglobulin and bikunin from AMBP_HUMAN, etc.) and polymorphic variants of proteins.

Quantitative performance was assessed through the consistency of the ratios of peptides matching to the same protein. The relative standard deviations of these peptide measurements indicated how accurate the protein measurement was (as an average of the matching peptide measurements). These procedures lowered the peptide relative standard deviations by a few percentage points.

Targeted Proteomics: Multiplexed Immunoassays

The concentrations of the 89 targeted proteins shown in Table 3 below were measured on a multiplexed immunoassay system (Rules Based Medicine, Austin, TX, USA) [McDade & Fulton, Device Diagn Ind 19:75-82, 1997; Fulton, et al., Clin Chem 43:1749-56, 1997]. The system performs a very large number of simultaneous reactions by using a flow cytometer and digital signal processor to perform real-time analysis of multiple microsphere-based assays. The three major components of the system are a benchtop flow cytometer, microspheres, and computer hardware and software. The volume of plasma used in these measurements was 50 microliters. The fluorescence signal recorded by the flow cytometer for each of the targeted proteins can be compared to standard curves for computation of the concentration of each of the targeted proteins. Quality control samples were included in the analysis.

Table 3: The 89 Proteins Measured in the Multiplex Immunoassay

| | | |
|---|---|---|
| 1. Adiponectin | 30. GST | 60. Lymphotactin |
| 2. Alpha-1 Antitrypsin | 31. G-CSF | 61. MDC |
| 3. Alpha-Fetoprotein | 32. GM-CSF | 62. MIP-1 alpha |
| 4. Alpha-2 Macroglobulin | 33. Growth Hormone | 63. MIP-1 beta |
| 5. Apolipoprotein A-I | 34. Haptoglobin | 64. MMP-2 |
| 6. Apolipoprotein C-III | 35. Immunoglobulin A | 65. MMP-3 |
| 7. Apolipoprotein H | 36. Immunoglobulin E | 66. MMP-9 |
| 8. Beta-2 Microglobulin | 37. Immunoglobulin M | 67. MCP-1 |
| 9. BDNF | 38. Insulin | 68. Myeloperoxidase |
| 10. C-Reactive Protein | 39. IGF-I | 69. Myoglobin |
| 11. Calcitonin | 40. ICAM- 1 | 70. PAI-1 |
| 12. Cancer Antigen 19-9 | 41. Interferon-gamma | 71. PAPP-A |
| 13. Cancer Antigen 125 | 42. Interleukin-1 alpha | 72. PSA, Free |
| 14. Carcinoembryonic Antigen | 43. Interleukin-1 beta | 73. Prostatic Acid Phosphatase |
| 15. CD40 | 44. Interleukin-1 ra | 74. RANTES |
| 16. CD40 Ligand | 45. Interleukin-2 | 75. Serum Amyloid P |
| 17. Complement 3 | 46. Interleukin-3 | 76. SGOT |
| 18. CK-MB | 47. Interleukin-4 | 77. Sex Hormone Binding Globulin |
| 19. Endothelin-1 | 48. Interleukin-5 | 78. Stem Cell Factor |
| 20. Eotaxin | 49. Interleukin-6 | 79. Thrombopoietin |
| 21. Epidermal Growth Factor | 50. Interleukin-7 | 80. Thyroid Binding Globulin |
| 22. ENA-78 | 51. Interleukin-8 | 81. Thyroid Stimulating Hormone |
| 23. Erythropoietin | 52. Interleukin-10 | 82. Tissue Factor |
| 24. ENRAGE | 53. Interleukin-12 p40 | 83. TIMP-1 |
| 25. Factor VII | 54. Interleukin-12 p70 | 84. Tumor Necrosis Factor-alpha |
| 26. Fatty Acid Binding Protein | 55. Interleukin-13 | 85. Tumor Necrosis Factor-beta |
| 27. Ferritin | 56. Interleukin-15 | 86. Tumor Necrosis Factor RII |
| 28. Fibrinogen | 57. Interleukin-16 | 87. VCAM- 1 |
| 29. FGF-basic | 58. Leptin | 88. VEGF |
| | 59. Lipoprotein (a) | 89. von Willebrand Factor |

The targeted protein analysis yielded useful information on the concentrations of 66 proteins across the set of samples from cases and controls. The dataset for these 66 proteins was used in univariate and multivariate analyses for this individual bioanalytical platform and was combined with the other bioanalytical platform datasets to generate the integrated dataset comprising 723 analytes.

Statistical Methods

Statistical analysis was carried out to address the primary objective of the study, namely to discover a molecular analyte (set), in blood or blood plasma and associated algorithm that predicts a near-term MACCE. Subjects who had the occurrence of MACCE during the two-year follow-up are denoted as disease cases and those with no occurrence of MACCE during follow-up as controls.

Statistical analysis included the following components:

1. Baseline characteristics of study subjects.
2. Principal Components Analysis (PCA) to visualize separation (if any) between the disease cases and controls from the data profiled by every platform.
3. Univariate models to detect individual analytes that are statistically significantly associated with the probability of near-term MACCE, after adjusting for conventional risk factors.
4. Multivariate models: Classifiers to obtain optimal subsets of analytes that are statistically significantly associated with the probability of near-term MACCE, with and without conventional risk factors. Receiver operating ccharacteristic (ROC) curves based on results from classifiers with and without conventional clinical factors were compared.

Baseline characteristics: Means and proportions for baseline characteristics were calculated for the disease cases and controls. The statistical significance of the differences in baseline characteristics between disease cases and controls was based on Wilcoxon rank-sum tests (continuous variables) and Chi-square tests (for proportions and RxC tables).

Principal Components Analysis: PCA score plots were generated for data obtained from each metabolomic and proteomic platform in the study. The motivations for these analyses include visualization of separation between the disease cases and controls, identification of outlier samples and for quality assessment of the datasets.

Univariate Methods: The primary univariate analysis was based on conditional logistic regression, taking into account the 1-to-1 matching of disease cases to controls. The univariate analysis consisted of regression models that evaluate the statistical significance of the association of each plasma analyte individually with outcome (disease case versus control status), after adjusting for relevant clinical covariates. Since the disease cases were matched to controls on age, race, gender and CAD Index, these variables do not appear in the regression model. The conditional logistic regression model adjusted for confounding factors that are not included in the matching strategy. The selection of conventional risk factors for inclusion into the model was based on a review of recent articles in medical literature reporting on results from studies investigating the effects of analytes with respective to cardiovascular outcomes. Each plasma analyte was modeled as quartiles based on the distribution of the analyte in the control subjects. Likelihood-ratio tests were used to assess the statistical significance of each analyte in predicting the outcome. Adjustment for multiple comparisons was based on methods by Storey (J. R. Statist. Soc. B 64: 479-498, 2002). Features with more than 50% missing values in either the disease cases or controls were excluded from this univariate analysis.

A second set of univariate analysis were conducted in which the analyte values were transformed to binary variables, where 0 indicates missing values and 1 otherwise. As in the previous univariate analysis, likelihood ratio tests were used to assess the statistical significance of each analyte in predicting outcome. Adjustment for multiple comparisons was based on methods by Storey (J. R. Statist. Soc. B 64: 479-498, 2002).

Graphical box-and-whisker plot representations for each statistically significant analyte, depicting disease cases and controls separately were also generated.

Plots of FDR adjusted p value compared to raw p value were generated. The distribution of p values seen in the plot were used as a basis for selecting a p-value threshold for selection of putative analytes in each platform. When data from all platforms was available, FDR adjusted p values were generated by integrating data from all platforms. A final univariate analyte list was generated from the integrated analysis. Selected results are listed in Table 4 below.

Table 4: Results of univariate analysis (Part 1).

| Platform | ID | Units | P value | Odds Ratio (OR$_4$) | Ratio of average concentration in cases to average concentration in controls |
|---|---|---|---|---|---|
| Polar LC/MS | Cysteine | arbitrary units (AU) | < 0.01 | 15.70 | 1.17 |
| Targeted Proteomics | von Willebrand Factor | micrograms/ml | < 0.01 | 17.67 | 1.44 |
| Targeted Proteomics | IL-8 | pg/ml | < 0.01 | 15.57 | 1.25 |
| Lipid LC/MS | 16:0/18:1 PC | AU | < 0.01 | 17.67 | 1.12 |
| GCMS | N-carboxy-alanine | AU | < 0.01 | 14.31 | 1.16 |
| Targeted Proteomics | Fibrinogen " | mg/ml | < 0.01 | 7.55 | 1.28 |
| Targeted Proteomics | MMP-2 | ng/ml | < 0.01 | 17.85 | 1.47 |
| Lipid LC/MS | 18:0/20:4 PE | AU | < 0.01 | 27.53 | 1.15 |
| Targeted Proteomics | Apolipoprotein AI | mg/ml | < 0.01 | 0.09 | 0.86 |
| Lipid LC/MS | 16:0/22:6 PE | AU | 0.01 | 14.87 | 1.08 |
| Lipid LC/MS | 18:1/18:0/18:0 TG | AU | 0.01 | 11.43 | 1.20 |
| Targeted Proteomics | Alpha-1 Antitrypsin | mg/ml | 0.01 | 5.59 | 1.12 |
| Lipid LC/MS | 18:2/18:1/17:0 TG | AU | 0.01 | 69.36 | 1.11 |
| Lipid LC/MS | 20:1/18:1/18:1 TG | AU | 0.02 | 12.64 | 1.27 |
| Lipid LC/MS | 16:0/16:0 PC | AU | 0.02 | 6.93 | 1.12 |
| Lipid LC/MS | 20:4 LPC | AU | 0.02 | 0.22 | 0.87 |
| Lipid LC/MS | 16:0 SM | AU | 0.02 | 7.91 | 1.08 |
| Targeted Proteomics | SHBG | nmol/ml | 0.03 | 6.81 | 1.25 |
| Lipid LC/MS | 18:1/17:1/16:0 TG | AU | 0.03 | 11.84 | 1.11 |
| GCMS | Arabinose | AU | 0.03 | 0.14 | 0.87 |
| Lipid LC/MS | 18:1/18:1/17:0 TG | AU | 0.03 | 10.68 | 1.16 |

Table 4B: Results of univariate analysis (Part 2).

| Platform | ID | Quartile 1 | Quartile 2 | Quartile 3 | Quartile 4 |
|---|---|---|---|---|---|
| Polar LC/MS | Cysteine | < 2041.1 | 2041.2-2699.2 | 2699.3-3675.9 | >3675.9 |
| Targeted Proteomics | von Willebrand Factor | < 35.2 | 35.2-47.7 | 47.8-69.0 | >69.0 |
| Targeted Proteomics | IL-8 | < 14.1 | 14.1-17.4 | 17.5-21.4 | >21.4 |
| Lipid LC/MS | 16:0/18:1 PC | <30075.8 | 30075.9-35320.5 | 35320.5-61392.4 | >61392.4 |
| GCMS | N-carboxyalanine | <3431.4 | 3431.4-3853.5 | 3853.6-4324.6 | >4324.6 |
| Targeted Proteomics | Fibrinogen | <3.5 | 3.5-4.3 | 4.3-5.2 | >5.2 |
| Targeted Proteomics | MMP-2 | <1037.5 | 1037.5-1520.0 | 1520.1-2157.5 | >2157.5 |
| Lipid LC/MS | 18:0/20:4 PE | <1861.8 | 1861.8-2138.4 | 2138.5-2429.0 | >2429.0 |
| Targeted Proteomics | Apolipoprotein AI | <0.30 | 0.30-0.36 | 0.37-0.46 | >0.46 |
| Lipid LC/MS | 16:0/22:6 PE | <1811.8 | 1811.8-2066.1 | 2066.2-2476.7 | >2476.7 |
| Lipid LC/MS | 18:1/18:0/18:0 TG | <15038.4 | 15038.4-17619.3 | 17619.4-22441.1 | >22441.1 |
| Targeted Proteomics | Alpha-1 Antitrypsin | <1.50 | 1.50-1.69 | 1.70-1.96 | >1.96 |
| Lipid LC/MS | 18:2/18:1/17:0 TG | <1963.7 | 1963.7-4369.5 | 4369.6-12667.6 | >12667.7 |
| Lipid LC/MS | 20:1/18:1/18:1 TG | <44210.6 | 44210.6-54354.2 | 54354.3-68063.3 | >68063.3 |
| Lipid LC/MS | 16:0/16:0 PC | <4859.1 | 4859.1-6019.0 | 6019.1-8158.0 | >8158.0 |
| Lipid LC/MS | 20:4 LPC | <4203.5 | 4203.5-5082.6 | 5082.7-6311.4 | >6311.4 |
| Lipid LC/MS | 16:0 SM | <4289.8 | 4298.8-5239.6 | 5239.7-6554.6 | >6554.6 |
| Targeted Proteomics | SHBG | <25.8 | 25.8-38.8 | 38.9-62.9 | >62.9 |
| Lipid LC/MS | 18:1/17:1/16:0 TG | <1649.8 | 1649.8-1861.9 | 1862.0-2189.9 | >2189.9 |
| GCMS | Arabinose | <26944.7 | 26944.7-33111.6 | 33111.7-43695.4 | >43695.4 |
| Lipid LC/MS | 18:1/18:1/17:0 TG | <1827.5 | 1827.5-2473.7 | 2473.8-3245.7 | >3245.7 |

In Table 4 above, PC means phosphatidylcholine, PE means phosphatidylethanolamine, TG means triacylglycerol, SM means sphingomyelin, LPC means lyso-phosphatidylcholine, IL means interleukin, and x:y denotes fatty acid chains containing x carbon atoms and y double bonds. Entries in the column denoted 'Odds Ratio' are the odds ratios corresponding to a comparison of subjects with analyte concentrations in the fourth quartile relative to subjects with analyte concentrations in the first quartile of the overall distribution of concentrations for the analyte across all subjects in the study. For analytes measured by mass spectrometry, the measurement units are indicated as 'arbitrary units', or 'AU'. Concentration measurements derived from the mass spectrometer represent processed ion counts as detected by the detector of the mass spectrometer instrument. For analytes measured by mass spectrometry, no absolute quantitation was performed.

The following fatty acid analytes are associated with risk for MACCE, where, respectively within the list of identified analytes, the first analyte has the strongest association with risk for MACCE and the last analyte has the weakest association with risk for MACCE: 18:2/18:1/17:0 TG, 18:0/20:4 PE, 16:0/18:1 PC, 16:0/22:6 PE, 20:4 LPC, 20:1/18:1/18:1 TG, 18:1/17:1/16:0 TG, 18:1/18:0/18:0 TG, 18:1/18:1/17:0 TG, 16:0 SM, and 16:0/16:0 PC.

For each of the fatty acid analytes identified in Table 4, except for 20:4 LPC, a higher concentration of the fatty acid can be more strongly associated with risk for MACCE (for example, subjects in the 4th quartile of the population distribution of 18:0/20:4 PE are more likely to suffer a MACCE than those in the lowest, i.e. 1st, quartile). For 20:4 LPC, it can be the opposite: subjects in the lowest, or 1st quartile of the population distribution of 20:4 LPC, are more likely to suffer a MACCE than those in the highest, i.e. 4th, quartile.

According to some embodiments, a clinician can identify a subject with an analyte measurement in the 4th quartile range of Table 4 above as an individual potentially at high risk for a MACCE in two years (with the exception of Apolipoprotein A1, 20:4 LPC, or Arabinose, which have odds ratios less than 1, meaning that subjects with values in the 1st quartile are at higher risk). According to another embodiment, a clinician can identify a subject with an analyte measurement within the 3rd or 4th quartile ranges of Table 4 above as an individual potentially at high risk for a MACCE in two years. According to a further embodiment, a clinician can identify a subject with an analyte measurement within the 2nd, 3rd or 4th quartile ranges of Table 4 above as an individual potentially at high risk for a MACCE in two years.

Multivariate Classification Methods

Supervised Multivariate Prediction Models: Multivariate predictive models were constructed in which the outcome to be predicted was the occurrence of MACCE within two years of index catheterization. Input variables to the model were (i) bioanalytical platform-specific plasma analytes, and (ii) plasma analytes as profiled by all bioanalytical platforms together. Each multivariable model was also built with and without adjusting for conventional clinical factors.

Multivariate classification procedure Random Forest (Breiman, Machine Learning 45:5-32, 2001) was applied to the data to obtain multivariate fingerprints predictive of MACCE. External cross-validation and label permutation testing was implemented in order to assess the statistical significance of the resulting models (Ambroise & McLachlan, Proc. Natl. Acad. Sci. 99:6562-2, 2002). The following outputs were available from each classifier:

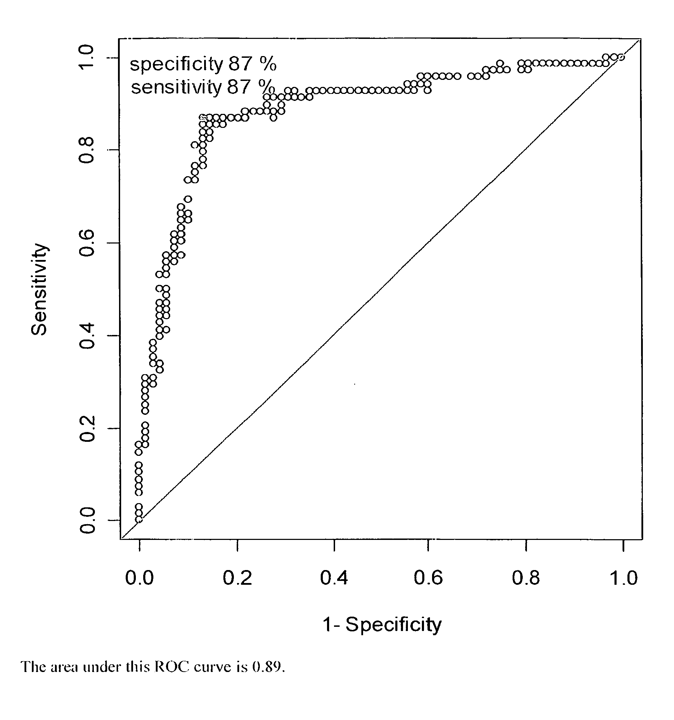

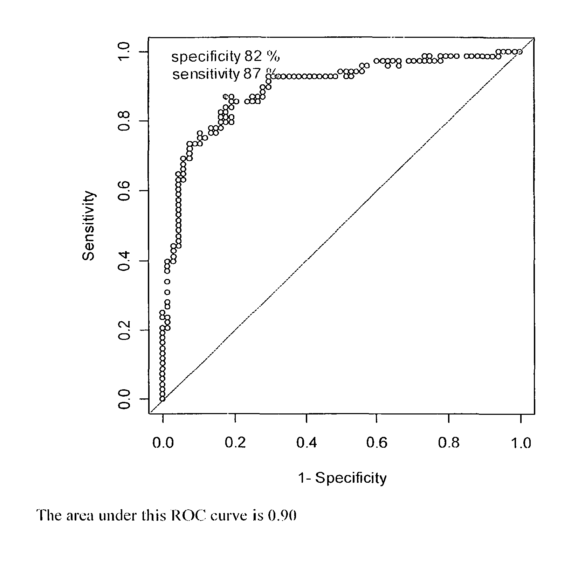

1. Predictive multivariate plasma analyte candidates for MACE prediction in the 'Disease Cases' and 'Controls' groups were reported with analytes ranked by importance in classification accuracy.
2. ROC curves of sensitivity versus specificity for each classifier (See Figures 1 and 2 for example)

Features with more than 50% missing values in either the disease cases or controls were excluded from multivariate analysis. Features with fewer than 50% missing values in disease cases and controls had missing values imputed using methods described Troyanskaya et al. (Bioinformatics 17(6) 520 - 525, 2001).

Analytes Identified According to Method

By using a multivariate statistical classifier analysis method (Random Forests - Breiman, Machine Learning 45:5-32, 2001) with the combined bioanalytical dataset of 723 analytes, we discovered sets of analytes and algorithms that can be used to predict the occurrence of a major adverse cardiovascular or cerebrovascular event in an individual within two years from the timepoint when a blood sample is obtained. Table 5 below presents the 50 most important analytes in their order or importance in the list of the total of 723 analytes in the Random Forests classifier based on one starting seed (Analysis #1). The partial analyte importance list from a Random Forests analysis and a recursive feature elimination approach to obtaining a classifier with just 50 analyte components using the same starting seed as Analysis #1 is shown in the column marker "Order of Importance from Analysis #2". The partial analyte importance lists from 3 additional Random Forests analyses and recursive feature elimination approaches to obtaining classifiers with just 50 analyte components derived from different starting seeds are shown in the columns for Analyses #3, #4 & #5. Irrespective of the starting seed for the Random Forests analysis or the use of recursive feature elimination to obtain a fixed 50 analyte component classifier, the most important 20 analytes are almost always within the top 50 ranked by importance from Analysis #1.

Table 5: 50 Most Important Analytes to Predict MACCE

| Analyte | Order of Importance from Analysis #1 | Order of Importance from Analysis #2 | Order of Importance from Analysis #3 | Order of Importance from Analysis #4 | Order of Importance from Analysis #5 |
|---|---|---|---|---|---|
| Tissue Factor | 1 | 3 | 3 | 2 | 3 |
| Cancer Antigen 125 | 2 | 2 | 1 | 3 | 2 |

(continued)

| Analyte | Order of Importance from Analysis #1 | Order of Importance from Analysis #2 | Order of Importance from Analysis #3 | Order of Importance from Analysis #4 | Order of Importance from Analysis #5 |
|---|---|---|---|---|---|
| Glutathione S-Transferase | 3 | 1 | 2 | 1 | 1 |
| Alpha-Fetoprotein | 4 | 4 | 4 | 4 | 4 |
| IPI00028413 (ITIH3) | 5 | 6 | 12 | 9 | 11 |
| IL-3 | 6 | 9 | 8 | 7 | 8 |
| 103_0at233 (Arabinose related fragment (M)) | 7 | 7 | 7 | 10 | 10 |
| von Willebrand Factor | 8 | 5 | 6 | 5 | 7 |
| IPI00011264 (CFHR1) | 9 | 12 | 15 | 12 | 12 |
| IL-8 | 10 | * | 10 | 11 | 14 |
| 178_1at107 (Cysteine M+H (M)) | 11 | 11 | * | 6 | 5 |
| 114_0at120 (Acetoacetate related fragment (M)) | 12 | 13 | 11 | 18 | 17 |
| Factor VII | 13 | | 27 | 23 | * |
| IPI00019755 (GSTO1) | 14 | 25 | 17 | 35 | 21 |
| 292_0at200 (Erythronic acid related fragment (M)) | 15 | 16 | 25 | * | 20 |
| IP100022417 (LRG1) | 16 | 30 | 37 | 15 | 15 |
| MMP-2 | 17 | 14 | 9 | 14 | 6 |
| Fibrinogen | 18 | 8 | 5 | 8 | 9 |
| TNF RII | 19 | 19 | 13 | * | * |
| TNF-beta | 20 | 10 | 16 | 21 | 16 |
| IPI00550991 (SERPINA3) | 21 | 20 | 29 | 19 | 18 |
| IPI00298971 (VTN) | 22 | 21 | 23 | 27 | * |
| IPI00641737 (HP) | 23 | * | 34 | 20 | 27 |

(continued)

| Analyte | Order of Importance from Analysis #1 | Order of Importance from Analysis #2 | Order of Importance from Analysis #3 | Order of Importance from Analysis #4 | Order of Importance from Analysis #5 |
|---|---|---|---|---|---|
| IPI00296176 (F9) | 24 | 46 | * | * | 32 |
| 202_0at311 (L-Tryptophan M+H (M)) | 25 | * | 30 | 36 | * |
| ICAM-1 | 26 | 28 | | 25 | 19 |
| 217_0at294 (Myo-inositol related fragment (M)) | 27 | * | 31 | * | * |
| 103_0at114 (Acetoacetate related fragment (M)) | 28 | * | 36 | 28 | 33 |
| 259_0at375 (unknown P7478_uk15 related fragment (M)) | 29 | * | * | * | 43 |
| IPI00167093 (CFHR1) | 30 | 48 | * | * | * |
| IPI00021885 (FGA) | 31 | * | * | * | * |
| IP100022429 (ORM1) | 32 | * | * | * | * |
| IPI00019943 (AFM) | 33 | 17 | 35 | 34 | 30 |
| 226_2at018 (Methylhistidine M+H (M)) | 34 | 44 | 39 | 44 | 31 |
| IPI00647915 (TAGLN2) | 35 | | | | |
| 191_0at110 (3-Hydroxybutanoic acid related fragment (M)) | 36 | * | * | * | * |
| 361_0at372 (Sucrose related fragment (M)) | 37 | * | * | * | * |
| 230_0at194 (L-4-Hydroxyproline related fragment (M)) | 38 | * | * | * | * |

(continued)

| Analyte | Order of Importance from Analysis #1 | Order of Importance from Analysis #2 | Order of Importance from Analysis #3 | Order of Importance from Analysis #4 | Order of Importance from Analysis #5 |
|---|---|---|---|---|---|
| 188_0at097 (Pyruvic acid related fragment (M)) | 39 | * | * | * | * |
| IPI00290283 (MASP1) | 40 | * | * | * | * |
| IPI00654888 (KLKB1) | 41 | 35 | * | * | * |
| IPI00020996 (IGFALS) | 42 | * | * | * | * |
| IP100029658 (EFEMP1) | 43 | 24 | 20 | 30 | 28 |
| 764_1213 (16: 0/22:6 PE M+H (M)) | 44 | 33 | * | 37 | * |
| IPI00745872 (ALB) | 45 | * | 18 | 17 | 26 |
| SGOT | 46 | 15 | 19 | 13 | 13 |
| 278_2at143 | 47 | * | * | * | * |
| IPI00745933 | 48 | * | * | * | * |
| * analyte was not a member of the top 50 analytes in the specific analysis | | | | | |

The full list of analytes from Random Forests analyses using four starting seeds are shown in the attached Appendices 1-4 along with the top 50 analytes from the Random Forests plus recursive feature elimination approach for the same four starting seeds in the attached Appendices 5-8.

By using the levels of the top 20 analytes from Analysis #1 in a plasma sample from an individual along with an algorithm, the prediction for that individual of the occurrence of a MACCE within two years would be made with 87% sensitivity and 87% specificity. This is illustrated by the Receiver Operating Characteristic (ROC) curve shown in Figure 1.

By using a multivariate statistical classifier analysis method (Random Forests - Breiman, Machine Learning 45:5-32, 2001) with the individual <u>targeted proteomics bioanalytical dataset of 66 analytes</u>, we discovered sets of analytes and algorithms that can be used to predict the occurrence of a heart attack in an individual within two years from the timepoint when a blood sample is obtained. Table 6 below presents the 10 most important analytes in one such set of analytes and their order or importance in a classifier derived from the multivariate analysis.

Table 6: 10 Most Important Analytes to Predict MACCE

| Protein Analyte | Variable Importance in Classifier |
|---|---|
| Glutathione S-Transferase | 0.048 |
| Cancer Antigen 125 | 0.045 |
| Tissue Factor | 0.04 |
| Alpha-Fetoprotein | 0.028 |
| von Willebrand Factor | 0.023 |
| Fibrinogen | 0.02 |
| SGOT | 0.015 |

(continued)

| Protein Analyte | Variable Importance in Classifier |
|---|---|
| IL-3 | 0.014 |
| IL-8 | 0.013 |
| CD40 | 0.008 |

By using the levels of these top 10 protein analytes in a plasma sample from an individual along with an algorithm, the prediction for that individual of the occurrence of a MACCE within two years would be made with 82% sensitivity and 87% specificity. This is illustrated by the Receiver Operating Characteristic (ROC) curve shown in Figure 2.

According to some embodiments, a MACCE index may be defined using ten (10) of the top analytes by the following linear equation:

$$y = c_1x_1 + c_2x_2 + c_3x_3 + c_4x_4 + c_5x_5 + c_6x_6 + c_7x_7 + c_8x_8 + c_9x_9 + c_{10}x_{10}$$

## [Equation 1]

where the values of $c_1$ through $c_{10}$ correspond to the values listed in Table 9 below, and the variables $x_1$ through $x_{10}$ represent the measurements of the analytes listed in Table 7 below. The analytes are measured as described in the <u>Targeted Proteomics: Multiplexed Immunoassays</u> section above. The measurement units for each analyte is listed in the table below. Prior to entering measurement values in to Equation 1, the values must be standardized. That is, from each measurement value was subtracted the average of all values derived from the entire study population (i.e. all case and control subjects), and subsequently the result was divided by the standard deviation of the analyte measurement derived from the entire study population (i.e. all case and control subjects). The corresponding values, namely the population averages and standard deviations, for the 10 analytes of Equation 1 are listed in Table 8.

Using such an equation as Equation 1, subjects with measurements of these ten analytes, each measurement standardized as described above using the appropriate values from Table 8, for whom the resulting value y in the equation above, the MACCE index, is greater than zero would be classified as being at risk of MACCE within two years, and otherwise would be classified as not being at risk for MACCE within two years.

Table 7: Equation 1, x variables.

| Variable in Equation 1 | Analyte | Measurement units |
|---|---|---|
| x1 | Alpha-Fetoprotein | nanograms / milliliter (ng/ml) |
| x2 | Cancer Antigen 125 | units / milliliter (U/ml) |
| x3 | CD40 | ng/ml |
| x4 | Fibrinogen | mg/ml |
| x5 | Glutathione S-Transferase | ng/ml |
| x6 | IL-3 | ng/ml |
| x7 | IL-8 | picograms / milliliter (pg/ml) |
| x8 | SGOT | micrograms per milliliter |
| x9 | Tissue Factor | ng/ml |
| X10 | von Willebrand Factor | micrograms per milliliter |

Table 8: Standard values.

| Analyte represented in Equation 1 | Population Average value (in measurement units of Table 7) | Standard Deviation (in measurement units of Table 7) |
|---|---|---|
| Alpha-Fetoprotein | 1.729 | 1.138 |
| Cancer Antigen 125 | 20.180 | 45.201 |

(continued)

| Analyte represented in Equation 1 | Population Average value (in measurement units of Table 7) | Standard Deviation (in measurement units of Table 7) |
|---|---|---|
| CD40 | 1.195 | 2.916 |
| Fibrinogen | 4.515 | 1.603 |
| Glutathione S-Transferase | 1.877 | 0.971 |
| IL-3 | 0.555 | 0.139 |
| IL-8 | 19.795 | 12.619 |
| SGOT | 26.108 | 7.069 |
| Tissue Factor | 3.392 | 2.135 |
| von Willebrand Factor | 54.738 | 29.320 |

Table 9: Equation 1, c variables.

| $c_1$ | -0.26243 |
|---|---|
| $c_2$ | 0.0947013 |
| $c_3$ | 0.00672228 |
| $c_4$ | -0.102292 |
| $c_5$ | 0.138434 |
| $c_6$ | -0.0191226 |
| $c_7$ | -0.121236 |
| $c_8$ | 0.0659886 |
| $c_9$ | 0.181278 |
| $c_{10}$ | -0.0900255 |

Yet another MACCE index may be defined using four (4) of the top analytes by the following linear equation:

$$y = c_1x_1 + c_2x_2 + c_3x_3 + c_4x_4 \qquad \textbf{[Equation 2]}$$

where the values of $c_1$ through $c_4$ take on those listed in Table 11 below, and the variables $x_1$ through $x_{10}$ represent the measurements of the analytes listed in Table 10 below. The analytes are measured as described in the Targeted Proteomics: Multiplexed Immunoassays section above. The measurement units for each analyte is listed in the table below. Prior to entering measurement values in to Equation 2, the values must be standardized. That is, from each measurement value was subtracted the average of all values derived from the entire study population (i.e. all case and control subjects), and subsequently the result was divided by the standard deviation of the analyte measurement derived from the entire study population (i.e. all case and control subjects). The corresponding values, namely the population averages and standard deviations, for the 4 analytes of Equation 2 are listed in Table 8 above.

Table 10: Equation 2, x variables.

| Variable in Equation 2 | Analyte | Measurement units |
|---|---|---|
| x1 | Alpha-Fetoprotein | nanograms / milliliter (ng/ml) |
| x2 | Cancer Antigen 125 | units / milliliter (U/ml) |
| x3 | Glutathione S-Transferase | ng/ml |
| x4 | Tissue Factor | ng/ml |

Table 11: Equation 2, c variables.

| c1 | -0.240085 |
|----|-----------|
| c2 | 0.0331361 |
| c3 | 0.17824 |
| c4 | 0.207721 |

Using such an equation as Equation 2, subjects with measurements of these four (4) analytes, each measurement standardized as described above using the appropriate values from Table 8, for whom the resulting value y in the equation above, the MACCE index, is greater than zero would be classified as being at risk of MACCE within two years, and otherwise would be classified as not being at risk for MACCE within two years.

An additional multivariate classification analysis method, Prediction Analysis of Microarrays (PAM, Tibshirani, et al., PNAS 99:6567-6572, 2002), was applied to the combined platforms dataset of 723 analytes. This analysis yielded optimal minimal analyte sets for classification of cases or controls. The analyte sets for four (4) PAM analyses using the same four seeds as those for Analyses #2, #3, #4, and #5, in the Random Forests cases above, are shown in the following Tables:

Table 12: Seed #1

| Seed #1 | | |
|---------|---|---|
| **Analyte** | **Scaled Delta Values** | |
| | **1. Case** | **2. Control** |
| Glutathione S-Transferase | 0.079 | -0.079 |
| Cancer Antigen 125 | 0.062 | -0.062 |
| von Willebrand Factor | -0.047 | 0.047 |
| Tissue Factor | 0.041 | -0.041 |
| Alpha-Fetoprotein | 0.036 | -0.036 |
| 178_1at107 (Cysteine M+H (M)) | -0.03 | 0.03 |
| MMP-2 | -0.023 | 0.023 |
| IL-8 | -0.013 | 0.013 |
| TNF-beta | 0.006 | -0.006 |
| TNF RII | -0.006 | 0.006 |
| Sensitivity = 83.8%; Specificity = 83.8% | | |

Table 13: Seed #2

| Seed #2 | | |
|---------|---|---|
| **Analyte** | **Scaled De Delta Values** | |
| | **1. Case** | **2. Control** |
| Glutathione S-Transferase | 0.079 | -0.079 |
| Cancer Antigen 125 | 0.062 | -0.062 |
| von Willebrand Factor | -0.047 | 0.047 |
| Tissue Factor | 0.041 | -0.041 |
| Alpha-Fetoprotein | 0.036 | -0.036 |
| 178_1at107 (Cysteine M+H (M)) | -0.03 | 0.03 |

(continued)

| Seed #2 | | |
|---|---|---|
| **Analyte** | **Scaled De Delta Values** | |
| | **1. Case** | **2. Control** |
| MMP-2 | -0.023 | 0.023 |
| IL-8 | -0.013 | 0.013 |
| TNF RII | -0.006 | 0.006 |
| TNF-beta | 0.006 | -0.006 |
| Sensitivity = 82.4%; Specificity = 80.9% | | |

Table 14: Seed #3

| Seed#3 | | |
|---|---|---|
| **Analyte** | **Scaled Delta Values** | |
| | **1. Case** | **2. Control** |
| Glutathione S-Transferase | -0.095 | 0.095 |
| Cancer Antigen 125 | -0.078 | 0.078 |
| von Willebrand Factor | 0.063 | -0.063 |
| Tissue Factor | -0.057 | 0.057 |
| Alpha-Fetoprotein | -0.052 | 0.052 |
| 178_1at107 (Cysteine M+H (M)) | 0.046 | -0.046 |
| MMP-2 | 0.039 | -0.039 |
| IL-8 | 0.029 | -0.029 |
| TNF-beta | -0.022 | 0.022 |
| TNF RII | 0.021 | -0.021 |
| 103_0at233 (Arabinose related fragment (M)) | 0.008 | -0.008 |
| IPI00022417 (LRG1) | 0.006 | -0.006 |
| IPI00745872 (ALB) | -0.005 | 0.005 |
| Sensitivity = 80.9%; Specificity = 80.9% | | |

Table 15: Seed #4

| Seed #4 | | |
|---|---|---|
| **Analyte** | **Scaled Delta Values** | |
| | **1. Case** | **2. Control** |
| Glutathione S-Transferase | -0.087 | 0.087 |
| Cancer Antigen 125 | -0.07 | 0.07 |
| von Willebrand Factor | 0.055 | -0.055 |
| Tissue Factor | -0.049 | 0.049 |
| Alpha-Fetoprotein | -0.044 | 0.044 |
| 178_1at107 (Cysteine M+H (M)) | 0.038 | -0.038 |

(continued)

| Seed #4 | | |
|---|---|---|
| **Analyte** | **Scaled Delta Values** | |
| | **1. Case** | **2. Control** |
| MMP-2 | 0.031 | -0.031 |
| IL-8 | 0.021 | -0.021 |
| TNF RII | 0.014 | -0.014 |
| TNF-beta | -0.014 | 0.014 |
| 103_0at233 (Arabinose related fragment (M)) | 0 | 0 |
| Sensitivity = 80.9%; Specificity = 82.4% | | |

This alternative multivariate classifier analysis by PAM yielded essentially the same analyte set for each of the different starting seeds and top-ranked analytes in order of importance consistent with, although not identical to, the results of the Random Forests analyses.

The present teachings include the identities of the sets of analytes that can be measured in a blood samples from an individual and used in conjunction with algorithms to calculate a MACCE index and to predict the occurrence of a MACCE within two years from the time of the blood sampling for that individual.

The present teachings also include the algorithms that can be used with information on the blood, blood plasma or blood serum levels of sets of analytes to calculate a MACCE index and to predict the occurrence of a MACCE within two years from the time of the blood sampling for that individual as described for analyte sets such as this by Breiman (Machine Learning 45:5-32, 2001).

**Claims**

1. A method of assessing the probability of a major adverse cardiovascular or cerebrovascular event in a human, the method comprising:

   measuring a concentration, in a blood-based sample of a human, of a set of analytes comprising alpha-fetoprotein, cancer antigen 125, glutathione S-transferase, and tissue factor;
   determining an index for the set of analytes; and
   identifying the human as having an increased likelihood of a major adverse cardiovascular or cerebrovascular event if the index is greater than zero, or a decreased likelihood of a major adverse cardiovascular or cerebrovascular event if the index is less than or equal to zero,
   wherein determining an index for the set of analytes comprises:

   standardizing the measured concentration of each analyte to obtain a standardized concentration;
   multiplying the standardized concentration of each analyte by an analyte constant to obtain an analyte value; and
   summing the analyte value of each analyte to obtain the index.

2. The method of any of claim 1, wherein the set of analytes further comprises CD40, fibrinogen, IL-3, IL-8, SGOT, and von Willebrand factor.

3. The method of claim 1 or 2, wherein standardizing the measured concentration comprises subtracting from the measured concentration a population average value to obtain a result and then dividing the result by a standard deviation of the population average value.

4. The method of any one of claims 1-3, wherein the blood-based sample comprises serum or plasma.

5. The method of any one of claims 1-4, further comprising transmitting, displaying, storing or outputting the index to a user interface device, a computer readable storage medium, or a local or remote computer system.

**Patentansprüche**

1. Verfahren zur Beurteilung der Wahrscheinlichkeit eines bedeutenden schädlichen kardiovaskulären oder zerebrovaskulären Ereignisses bei einem Menschen, wobei das Verfahren umfasst:

   Messen einer Konzentration, in einer auf Blut basierenden Probe eines Menschens, einer Reihe von Analyten, umfassend Alpha-Fetoprotein, Krebsantigen 125, Glutahion-S-Transferase und Gewebefaktor;
   Bestimmen eines Index für die Reihe von Analyten; und
   Identifizieren des Menschen als jemanden, der eine erhöhte Wahrscheinlichkeit für ein bedeutendes schädliches kardiovaskuläres oder zerebrovaskuläres Ereignis aufweist, wenn der Index größer als Null ist, oder als jemanden, der eine verringerte Wahrscheinlichkeit für ein bedeutendes schädliches kardiovaskuläres oder zerebrovaskuläres Ereignis aufweist, wenn der Index geringer als oder gleich Null ist,
   wobei das Bestimmen des Index für die Reihe von Analyten umfasst:

      Standardisieren der gemessenen Konzentration jedes Analyts, um eine standardisierte Konzentration zu erhalten;
      Vervielfältigen der standardisierten Konzentration jedes Analyts durch eine Analytkonstante, um einen Analytwert zu erhalten; und
      Addieren des Analytwerts jedes Analyts, um den Index zu erhalten.

2. Verfahren gemäß Anspruch 1, wobei die Reihe von Analyten des Weiteren CD40, Fibrinogen, IL-3, IL-8, SGOT und von-Willebrand-Faktor umfasst.

3. Verfahren gemäß Anspruch 1 oder 2, wobei das Standardisieren der gemessenen Konzentration Subtrahieren eines Populationsdurchschnittswerts von der gemessenen Konzentration umfasst, um ein Ergebnis zu erhalten, und dann Dividieren des Ergebnisses durch eine Standardabweichung des Populationsdurchschnittswerts.

4. Verfahren gemäß einem der Ansprüche 1-3, wobei die auf Blut basierende Probe Serum oder Plasma enthält.

5. Verfahren gemäß einem der Ansprüche 1-4, welches des Weiteren übermitteln, anzeigen, speichern oder ausgeben des Index an eine Benutzerschnittstellenvorrichtung, ein computerlesbares Speichermedium oder ein lokales oder Ferncomputersystem umfasst.

**Revendications**

1. Procédé d'estimation de la probabilité d'un événement cardiovasculaire ou cérébrovasculaire préjudiciable majeur chez un humain, le procédé comprenant les étapes consistant à :

   mesurer une concentration, dans un échantillon à base de sang d'un humain, d'un jeu d'analytes, comprenant l'alpha-fétoprotéine, l'antigène CA125, la glutathione S-transférase et un facteur tissulaire ;
   déterminer un indice pour le jeu d'analytes ; et
   identifier l'humain comme ayant une vraisemblance accrue d'un événement cardiovasculaire ou cérébrovasculaire préjudiciable majeur si l'indice est supérieur à zéro, ou une vraisemblance réduite d'un événement cardiovasculaire ou cérébrovasculaire préjudiciable majeur si l'indice est inférieur ou égal à zéro,
   où la détermination d'un indice pour le jeu d'analytes comprend les sous-étapes consistant à :

      normaliser la concentration mesurée de chaque analyte pour obtenir une concentration normalisée ;
      multiplier la concentration normalisée de chaque analyte par une constante d'analyte pour obtenir une valeur d'analyte ; et
      sommer la valeur d'analyte de chaque analyte pour obtenir l'indice.

2. Procédé selon la revendication 1, dans lequel le jeu d'analytes comprend en outre CD40, le fibrinogène, IL-3, IL-8, SGOT, et le facteur de von Willebrand.

3. Procédé selon la revendication 1 ou 2, dans lequel la normalisation de la concentration mesurée comprend les sous-étapes consistant à soustraire de la concentration mesurée une valeur moyenne de population pour obtenir un résultat puis diviser le résultat par un écart-type de la valeur moyenne de la population.

4. Procédé selon l'une quelconque des revendications 1 à 3, dans lequel l'échantillon à base de sang comprend du sérum ou du plasma.

5. Procédé selon l'une quelconque des revendications 1 à 4, comprenant en outre l'étape consistant à transmettre, afficher, stocker ou fournir l'indice à un dispositif d'interface utilisateur, un support de stockage lisible par ordinateur, ou un système d'ordinateur local ou éloigné.

The area under this ROC curve is 0.89.

The area under this ROC curve is 0.90

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2007003981 A
- US 5091513 A
- US 5132405 A
- US 4704692 A
- US 4946778 A

### Non-patent literature cited in the description

- **Naghavi et al.** *Circulation,* vol. 108, 1664-72
- *CIRCULATION,* 2003, vol. 108, 1772-8
- **Wilson et al.** *Circulation,* 1998, vol. 97, 1837-47
- **ATP III.** *JAMA,* 2001, vol. 285, 2486-97
- **Khot et al.** *JAMA,* 2003, vol. 290, 898-904
- **Greenland et al.** *JAMA,* 2003, vol. 290, 891-7
- **Vasan.** *Circulation,* 2006, vol. 113, 2335-62
- **Hagberg.** *NMR Biomed.,* 1998, vol. 11, 148-56
- **Stordeur et al.** *J. Immunol. Methods,* 2002, vol. 259, 55-64
- **Tan et al.** *Proc. Nat'1 Acad. Sci. USA,* 2002, vol. 99, 11387-11392
- **O'Farrell.** *J. Biol. Chem.,* 1975, vol. 250, 4007-4021
- **Butt, W.R.** Practical Immunology. Marcel Dekker, 1984
- **Harlow et al.** Antibodies, A Laboratory Approach. 1988
- **Clackson et al.** *Nature,* 1991, vol. 352, 624-628
- **McDade ; Fulton.** *Device Diagn Ind,* 1997, vol. 19, 75-82
- **Fulton et al.** *Clin Chem,* 1997, vol. 43, 1749-56
- **Storey.** *J. R. Statist. Soc. B,* 2002, vol. 64, 479-498
- **Breiman.** *Machine Learning,* 2001, vol. 45, 5-32
- **Ambroise ; McLachlan.** *Proc. Natl. Acad. Sci.,* 2002, vol. 99, 6562-2
- **Troyanskaya et al.** *Bioinformatics,* 2001, vol. 17 (6), 520-525
- *Random Forests - Breiman, Machine Learning,* 2001, vol. 45, 5-32
- **PAM, Tibshirani et al.** *PNAS,* 2002, vol. 99, 6567-6572